# EUROPEAN PATENT SPECIFICATION

(11) **EP 4 157 246 B1**
(45) Date of publication and mention of the grant of the patent: **28.05.2025**
(21) Application number: 21718921.6
(22) Date of filing: 21.04.2021
(51) Int. Cl.: A61K 31/138, A61K 31/519, A61K 31/565, A61K 45/06, A61P 35/00

(54) **THERAPEUTIC COMBINATION FOR THE TREATMENT OF BREAST CANCER**
THERAPEUTISCHE KOMBINATION ZUR BEHANDLUNG VON BRUSTKREBS
ASSOCIATION THÉRAPEUTIQUE POUR LE TRAITEMENT DU CANCER DU SEIN

(30) Priority: 23.04.2020 IT 202000008710
(43) Date of publication of application: 05.04.2023
(73) Proprietor: IFOM - Instituto Fondazione Di Oncologia Molecolare ETS In Breve IFOM ETS, 20139 Milano (IT); University of Southern California, Los Angeles, CA 90089 (US); Università degli Studi di Genova, 16126 Genova (IT)
(72) Inventor: NENCIONI, Alessio, 16132 Genova (IT); CAFFA, Irene, 17021 Alassio (SV) (IT); LONGO, Valter, Los Angeles, California 90015 (US); SPAGNOLO, Vanessa, 20137 Milano (IT)
(74) Representative: Botti & Ferrari S.p.A.
(86) International application number: PCT/EP2021/060341
(87) International publication number: WO 2021/214119

(56) References cited:
- WO-A1-2017/140641
- CRISTOFANILLI MASSIMO ET AL: "Fulvestrant plus palbociclib versus fulvestrant plus placebo for treatment of hormone-receptor-positive, HER2-negative metastatic breast cancer that progressed on previous endocrine therapy (PALOMA-3): final analysis of the multicentre, double-blind, phase 3 randomised controlled trial", THE LANCET ONCOLOGY, ELSEVIER, AMSTERDAM, NL, vol. 17, no. 4, 3 March 2016 (2016-03-03), pages 425 - 439, XP029483315, ISSN: 1470-2045, DOI: 10.1016/S1470-2045(15)00613-0
- NENCIONI ALESSIO ET AL: "Fasting and cancer: molecular mechanisms and clinical application", NATURE REVIEWS CANCER, NATURE PUB. GROUP, LONDON, vol. 18, no. 11, 16 October 2018 (2018-10-16), pages 707 - 719, XP036668994, ISSN: 1474-175X, [retrieved on 20181016], DOI: 10.1038/S41568-018-0061-0
- CAFFA IRENE ET AL: "Fasting-mimicking diet and hormone therapy induce breast cancer regression", NATURE, vol. 583, no. 7817, 15 July 2020 (2020-07-15), pages 620 - 624, XP037311058, ISSN: 0028-0836, DOI: 10.1038/S41586-020-2502-7

## Description

### Technical Field

The present invention concerns the technical field of the pharmaceutical industry.

In particular, the invention relates to a therapeutic combination of a compound having antiestrogenic activity, in particular a compound selected among selective estrogen receptor modulators (SERMs), selective estrogen receptor degraders (SERDs) and aromatase inhibitors (AIs), and a CDK4/6 inhibitor, for use in the treatment of breast cancer (BC) and other estrogen sensitive cancers in association with and a defined dietetic regimen as defined in the claims.

### Prior art

Breast cancer (BC) is the most frequently diagnosed malignancy with 1.7 million new diagnoses/year (23% of cancer diagnoses)¹. About 20% of BC recur after curative surgery; moreover, about 10% of all patients present with metastatic breast cancer (mBC) at diagnosis; mBC remains essentially incurable. Approximately 75% of BCs express estrogen receptor (ER) and/or progesterone receptor (PgR). Recently, the introduction of the CDK4/6 inhibitors (CDK4/6inh) palbociclib (PALB), ribociclib or abemaciclib in combination with standard endocrine therapy (ET) has improved progression free survival (PFS) and tumor response rates (RRs) in patients with HR+HER2-mBC². Despite these improvements, HR+HER2- mBC eventually progresses even under ET+CDK4/6inh [PFS is approximately 24 months in the first-line setting and is about 9 months in endocrine-refractory mBC] and most patients with HR+ mBC still die of their disease.

Therefore, new strategies are needed to enhance the activity of ET, reducing the chances of patient relapse, and to prolong survival and, hopefully, cancer free survival in patients with HR+ mBC.

Periodic cycles of water fasting/fasting mimicking diet (FMD) have antitumor effects in mouse tumor models and synergize with chemotherapy, radiotherapy, and tyrosine kinase inhibitors³. The anticancer properties of fasting/FMD reflect the inability of malignant cells to adapt to rapid nutrient and growth factor deprivation, especially in combination with other therapies.

Moreover, the FMD stimulates tumor infiltration by cytotoxic CD8+ T lymphocytes, while reducing immunosuppressive regulatory T cells. Fasting/FMDs also have the added value of protecting healthy tissues from anticancer treatment-induced toxicity and can induce multisystem pro-regenerative effects⁴. Clinical studies of fasting/FMD in patients undergoing chemotherapy support its feasibility and safety and, consistent with the mouse data on the effect of fasting/FMDs on chemotherapy tolerability, cycles of FMD were recently reported to help patients maintain their Health Related Quality of Life (HRQoL) and avoid fatigue while receiving chemotherapy for ovarian cancer or BC treatment^{3,5}.

In patent application WO 2017/140641, the present Applicants disclosed a method of treating breast cancer or other estrogen-responsive tumors and cancer in a human patient, wherein the method comprises subjecting the patient to reduced calorie intake for a period of 24-190 hours, while the patient is being treated with a compound having antiestrogenic activity, in particular a SERM, a SERD or an AI.

The Applicants have now surprisingly found that FMD, by virtue of its capability of reducing the circulating growth factors, in particular insulin, Igf-1 and leptin, allows to further increase the progression free survival (PFS) and to induce tumor regressions in mouse models of HR+/HER2- BC when combined with an antiestrogenic compound and with a CDK4/6 inhibitor. In line with these findings in the mouse, very promising results with this approach (combined FMD, antiestrogenic compound and CDK4/6 inhibitors) were obtained in patients enrolled in either one of two clinical trials (NCT03595540, NCT03340935).

### Summary of the invention

In an aspect thereof, the present invention relates to a therapeutic combination of a compound having antiestrogenic activity and a CDK4/6 inhibitor for use in a method for the treatment of breast cancer (BC) or other estrogen-responsive tumors in a human patient, wherein the method comprises subjecting said patient to reduced calorie intake for a period of 24-190 hours while said patient is being treated with said therapeutic combination, wherein said compound having antiestrogenic activity is fulvestrant and said CDK4/6 inhibitor is palbociclib, and wherein said reduced calorie intake is a daily calorie intake reduced by 50-100%, more preferably by 75-100%, with respect to the normal daily calorie intake, which is 2000-3000 kcal/day for an adult male subject and 1600-2400 kcal/day for an adult female subject.

In a non-claimed aspect, the above-mentioned compound having antiestrogenic activity is preferably selected among the group consisting of SERMs, SERDs, and of AIs.

In a non-claimed aspect, by reduced calorie intake it is hereby meant a daily calorie intake reduced by 10-100%, with respect to the normal calorie intake, including total starvation.

The subject's normal calorie intake is the number of kcal that the subject consumes to maintain his/her weight. The subject's normal calorie intake may be estimated by interviewing the subject or by consideration of a subject's weight. As a rough guide, subject's normal calorie intake is on average 2000-3000 kcal/day for men and 1600-2400 kcal/day for women.

Preferably, when the daily calorie intake is reduced , the patient is fed with foods with a high content of monounsaturated and polyunsaturated fats and a reduced content of proteins and sugars (≥ 40% of calories coming from fat). This because a diet based on such foods has beneficial effects that are similar to those of starvation.

Preferably said period of reduced calorie intake ranges from 48 to 150 hours, and most preferably it is of about 120 hours.

Among the SERMs that can be used, the following are mentioned: tamoxifen, raloxifene, toremifene, lasofoxifene, ospemifene, arzoxifene, bazedoxifene.

Among the SERDs that can be used, the following are mentioned: fulvestrant, RU 58668, GW7604, GDC-0810, AZD9496, pipendoxifene, acolbifene, OP-1074 ((2S)-3-(4-hydroxyphenyl)-4-methyl-2-(4-{2-[(3R)-3-methylpyrrolidin-1-yl]ethoxy}phenyl)-2H-chromen-7-ol).

Fulvestrant is the SERD for use in the method of the present invention.

Among the AIs that can be used, the following are mentioned: anastrozole, exemestane, letrozole, vorozole, formestane, fadrozole.

Among the CDK4/6 inhibitors that can be used in a non-claimed aspect, the following are mentioned: ribociclib, aminociclib, abemaciclib, trilaciclib, voruciclib, purvalanol A and olomoucine II. Palbociclib is the CDK4/6 inhibitor for use in the present invention.

In a particular aspect, the present invention relates to a therapeutic combination of fulvestrant and palbociclib for use in a method for the treatment of BC or other estrogen-responsive tumors and cancers in a human patient, wherein the method comprises subjecting said patient to reduced calorie intake for a period of 24-190 as defined in the claims while said patient is being treated with said therapeutic combination.

As used herein, "cancer" refers to a disease or disorder characterized by uncontrolled division of cells and the ability of these cells to spread, either by direct growth into adjacent tissue through invasion, or by implantation into distant sites by metastasis. The present invention is focused on the treatment of BC, in particular ER-positive BC but it applies to all estrogen-responsive tumors and cancers.

The above-mentioned period of reduced calorie intake with concurrent administration of the compound having antiestrogenic activity to the patient can be repeated one or more times after respective periods of 5-60 days, during which the patient is given the antiestrogenic compound and the CDK4/6 inhibitor while following a diet involving a normal calorie intake.

Such reduced calorie intake can be obtained by means of dietetic foods with reduced calorie, sugar, and protein content, but containing relatively high levels of unsaturated "good fats" and all necessary micronutrients to prevent malnutrition.

In another aspect, the present invention relates to a pharmaceutical composition comprising a compound with antiestrogenic activity as defined above, a CDK4/6 inhibitor as defined above and a pharmaceutically acceptable carrier for use in the method for the treatment of BC and other estrogen-dependent tumors and cancers in a patient as defined above.

As it will become clear from the experimental results reported in the following sections, it has unexpectedly been found that starvation and also a reduced calorie intake for periods of 24-72, positively affect the anticancer efficacy of a concurrent anticancer treatment with a therapeutic combination of compound having antiestrogenic activity and a CDK4/6 inhibitor.

A positive effect on the efficacy of a concurrent anticancer treatment with a therapeutic combination of a compound having antiestrogenic activity and a CDK4/6 inhibitor is also obtained when the above mentioned periods of reduced calorie intake are replaced by corresponding periods of normal calorie intake, during which the patients are only fed with the above-mentioned foods with a high content of monounsaturated and polyunsaturated fats and a reduced content of proteins and sugars (≥ 40% of calories coming from fat), since a diet based on such foods has beneficial effects that are similar to those of starvation.

In a non-claimed aspect, the present disclosure thus also concerns a therapeutic combination of a compound having antiestrogenic activity and a CDK4/6 inhibitor for use in a method for the treatment of cancer in a human patient, wherein the method comprises feeding said patient only with foods with a high content of monounsaturated and polyunsaturated fats and a reduced content of proteins and carbohydrates (≥ 40% of calories coming from fat), in such an amount as to ensure a normal daily calorie intake, i.e. 2000-3000 kcal/day for an adult male subject and 1600-2400 kcal/day for an adult female subject, for a period of 24-190 hours, while said patients is being treated with said therapeutic combination.

The compounds and compositions according to the invention may be administered with any available and efficient delivery system, comprising, but not limited to, oral, buccal, parenteral, inhalatory routes, topical application, by injection, by transdermic or rectal route (for ex. by means of suppositories) in dosage unit formulations containing conventional, pharmaceutically acceptable and non-toxic carriers, adjuvants and vehicles. The administration by parenteral route comprises subcutaneous, intravenous, intramuscular, intrasternal injection or infusion techniques.

The solid dosage forms for the administration by oral route comprise, for example, capsules, tablets, powders, granules and gels. In such solid dosage forms, the active compound may be mixed with at least one inert diluent such as, for example, sucrose, lactose or starch. These dosage forms normally also comprise additional substances different from the inert diluents, such as, for example, lubricating agents like magnesium stearate.

The injectable preparations, for example aqueous or oily sterile injectable solutions or suspensions, may be formulated according to the known technique and by optionally using appropriate dispersing, wetting and/or suspending agents.

The pharmaceutical preparations according to the present invention may be produced by using conventional pharmaceutical techniques, as described in the various pharmacopoeias or handbooks of the field such as, for example, "Remington's Pharmaceutical Sciences Handbook", Mack Publishing, New York, 18th Ed., 1990.

The average daily dosage of the compounds according to the present invention depends on many factors, such as, for example, the seriousness of the disease and the conditions of the patient (age, weight, sex): The dose may generally vary from 1 mg to 1500 mg per day of compound according to the invention, optionally divided into more administrations.

The present invention will be further described with reference to the appended drawings and to certain embodiments, which are provided here below by way of illustration and not of limitation.

### Brief description of the drawings

Figs. 1a-c are three diagrams showing the growth of MCF7, ZR-75-1 and T47D xenografts treated with TMX/FULV, weekly 48h fasting/FMD or combined ET and fasting/FMD (n=6/8).
Fig. 1d comprises four micrographs of tumor-derived organoids from patients with HR+BC, which have been treated with tamoxifen with or without short-term starvation (STS) (culture conditions 1%FBs and 0.5 g/L glucose) before being imaged and before viability quantification. The viability quantification is illustrated by the three histograms on the right side of Fig. 1d.
Fig. 1e comprises two diagrams showing (left side) the MCF7 xenograft growth and (rights side) the corresponding mouse survival in response to prolonged TMX administration, weekly FMD, or their combination (n=7/10).
Fig. 1f is a histogram showing the levels of β-hydroxybutyrate in mice treated with 48h fasting/FMD (or *ad lib* diet) with or without TMX/FULV (n=6 mice/group.
Fig. 1g consists of five histograms showing, from left to right, the levels of IGF1, IGFBP1, IGFBP3, leptin and c-peptide in mice treated with 48h fasting/FMD (or *ad lib* diet) with or without TMX/FULV (n=6 mice/group.

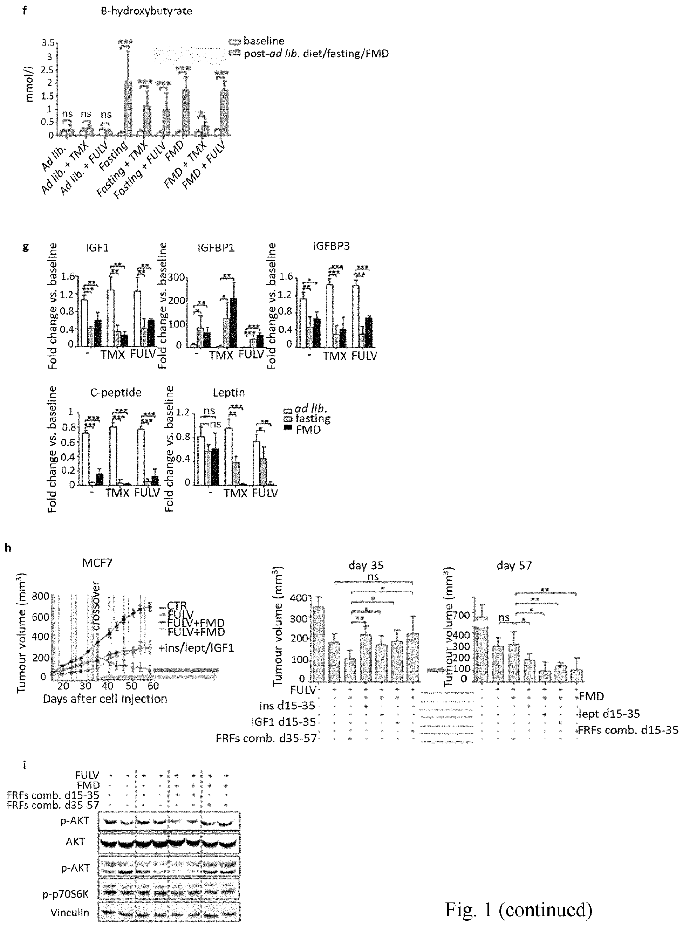

Figs. 1h-i include three diagrams and an immunoblotting showing the growth of MCF7 xenografts in mice treated with or without FULV, FULV plus weekly FMD, or combined FULV, FMD and i.p. insulin, IGF1, leptin, or the three FRFs combined (n=6-9 mice/group). At day 35, FRF administration was withdrawn, while it was started in mice that had only received FULV plus FMD. Mice were sacrificed at the end of the last FMD cycle and phosphorylated and total AKT and p70S6K in the tumors were detected by Western blotting (Fig. 1i). Abbreviations: ns: non-significant; *: p<0.05; **: p<0.01; ***: p<0.001. All animal experiments were performed in six-to-eight week-old female BALB/c athymic mice (nu+/nu+).
Fig. 2a consists of two immunoblottings showing PI3K/AKT/mTOR signaling and EGR1 and PTEN levels in MCF7 cells treated with (left side) FULV, STS/48h FMD or their combination and with (right side) TMX, STS/48h FMD or their combination.
Fig. 2b is an immunoblotting showing PI3K/AKT/mTOR signaling and EGR1 and PTEN levels in MCF7 xenograft-bearing mice (n=6/8), which were treated for four weeks. Mice were sacrificed and tumour masses were isolated at the end of the last FMD cycle.
Fig. 2c is a histogram showing *EGR1* and *PTEN* expression in HR+ BC organoids treated with TMX, STS or their combination
Fig. 2d includes a histogram and an immunoblotting showing the cell viability in MCF7 cells in response to EGR1 silencing (shRNA#1), TMX, FULV, STS, or their combination.
Fig. 2e is an immunoblotting showing PTEN levels and AKT phosphorylation in MCF7 cells in response to EGR1 silencing (shRNA#1), TMX, FULV, STS, or their combination.
Fig. 2f is a histogram showing the viability of control and EGR1-silenced (shRNA#1) MCF7 in response to TMX, STS, GDC0068, AZD5363, LY294002, or their combinations.
Fig. 2g includes a histogram and an immunoblotting showing MCF7 cell viability in response to myr-AKT overexpression, TMX, FULV, STS, or their combination.
Fig. 2h is a diagram showing the *in vivo* growth of MCF7 with silenced EGR1 (shRNA#1) or PTEN, or with myr-AKT overexpression in response to FULV plus weekly FMD (n=8 mice/group).
Fig. 2i is an immunoblotting showing EGR1, PTEN and 4E-BP1 levels in MCF7 treated w/ or w/o STS plus TMX, the combination of insulin, IGF1 and leptin, or 17β-estradiol.
Fig. 2l is an immunoblotting showing EGR1 and GAPDH levels in MCF7 w/ or w/o myr-AKT overexpression, treated w/ or w/o FULV, TMX, STS, or their combinations.
Figs. 2m and 2n are histograms showing ER transcriptional activity and ER target gene expression in MCF7 cells in response to TMX, FULV, STS, or their combinations. Abbreviations: ns: non-significant; *: p<0.05; **: p<0.01; ***: p<0.001. Animal experiments were performed in in six-to-eight week-old female BALB/c athymic mice (nu+/nu+).

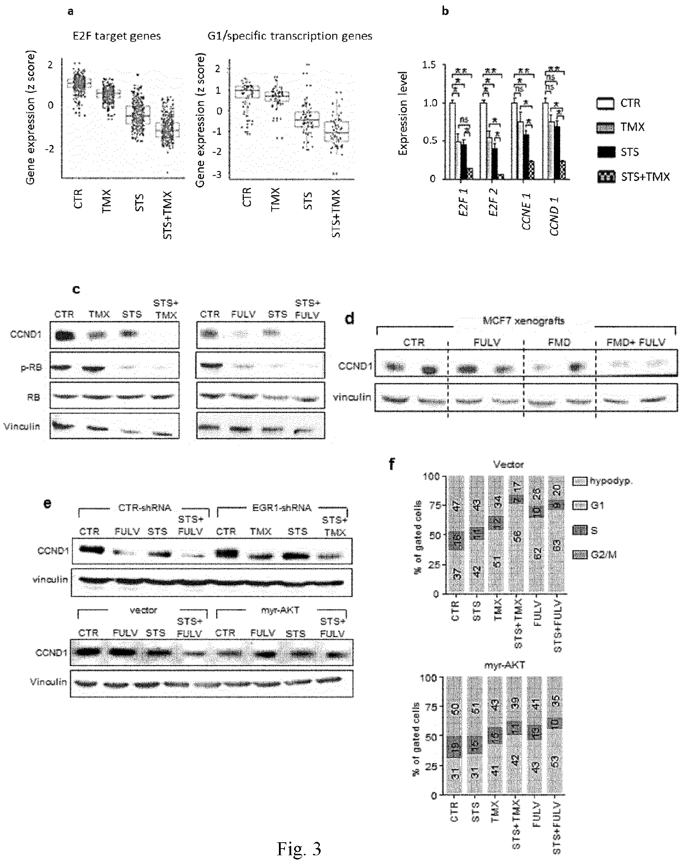

Figs. 3a-d show G1/S transition-promoting gene down-regulation with combined ET and STS in MCF7 cells as shown by gene set enrichment analysis (GSEA), quantitative polymerase chain reaction (qPCR) and western blotting (WB) *in vitro* and *in vivo.* In c, the effect of TMX, FULV, STS on CCND1 and RB phosphorylation was detected by Western blotting in MCF7 cells. In d, Western blotting was used to detect the effect of FULV, FMD or their combination on CCND1 expression in MCF7 xenografts. MCF7 cells were injected into six-to-eight-week-old female BALB/c athymic mice (nu+/nu+); in all treatment groups (n=6/8), mice were sacrificed and tumour masses were isolated at the end of the last FMD cycle
Fig. 3e is an immunoblotting showing the effect of EGR1 silencing, myr-AKT overexpression, FULV, TMX, STS, or their combinations on CCND1 expression in MCF7 cells.
Fig. 3f includes two diagrams showing a cell-cycle analysis of control and myr-AKT-overexpressing MCF7 treated with ET, STS, or their combinations.
Fig. 3g is a drawing showing a putative model for the cooperation between fasting/FMD and ET at the level of PI3K-AKT-mTOR signaling and of CCND1 regulation.
Fig. 3h includes two diagrams showing the growth of orthotopic MCF7-xenograft and the progression-free survival in six-to-eight week-old female NOD/SCID mice treated with FULV, FMD, PALB or their combinations (n=15-18 mice/arm).
Fig. 3i includes three diagrams showing the *in vivo* response to weekly FMD of MCF7 xenografts with acquired resistance to FLUV plus PALB (left panel) and the response of re-transplanted, resistant MCF7 cells and of parental MCF7 to combined FULV and PALB w/ or w/o weekly FMD (middle and right panel). Abbreviations: ns: non-significant; *: p<0.05; **: p<0.01; ***: p<0.001.
Figs 4a-f show body weight, hand grip, phase angle, fat-free and fat mass (n=23), and muscle area (highlighted in red) quantification at L3 (pt.#1) and at the Louis angle (pt.#3) level in patients with HR+ BC treated with ET and cyclic FMD (NCT03595540).
Fig. 4g includes PET scans from a patient with metastatic HR+/HER2-BC (#26) exhibiting a complete metabolic response to combined FULV, PALB and FMD.
Figs. 4h and 4i include 12 diagrams showing serum IGF1, IGFBP1, IFGBP3, c-peptide, leptin, blood ketone bodies (h) and glucose (h) before and after a FMD cycle (h; n=6), and before and three weeks after a FMD cycle (i; n=23) in patients treated with ET and FMD for HR+ BC (NCT03595540).
Fig. 4l is a histogram showing leptin serum levels in mice treated with FULV/TMX, weekly FMD or their combinations for two weeks. Serum was collected one week after the last FMD cycle (n=6).
Fig. 4m is a diagram showing MCF7 xenograft growth (n=6/8) after a one-month treatment w/ or w/o TMX/FULV, weekly 48h FMD, or their combinations.
Figs. 4n and 4o are respective diagrams showing MCF7 engraftment and growth in mice (n=6) pre-treated for one month with TMX, FULV, FMD or their combinations (MCF7 cells were inoculated one week after the end of pre-treatment). Abbreviations: ns: non-significant; *: p<0.05; **: p<0.01; ***: p<0.001. Animal experiments were performed in female BALB/c athymic mice (nu+/nu+).
Figs. 5a-e show viability and colony formation assays in MCF7, T47D and ZR-75-1 cells treated w/ or w/o ET and STS.
Fig. 5f includes 24 photos (left side) and two histograms (right side) showing tumor images (left) and weights (right) taken from MCF7 or ZR-75-1 xenograft-bearing mice which were treated for three weeks with TMX, FULV, weekly 48h FMD or combined ET and FMD (n=6/8). Mice from all treatment groups were sacrificed at the end of the last FMD cycle. Tumors were imaged (left, MCF7) and weighted (right).
Fig. 5g includes two diagrams showing mouse weight during treatment with TMX/FULV, fasting/FMD or their combinations in MCF7 xenograft- or ZR-75-1 xenograft-bearing mice.
Fig. 5h includes four diagrams showing the effect of glucose add-back in HR+ BC cell lines treated with TMX, FULV, STS and their combinations.
Fig. 5i includes three histograms showing serum adiponectin, TNFα and IL1β concentration in mice before and after a 48h fasting/FMD (or ad lib. diet) w/ or w/o treatment with TMX/FULV (n=6).
Fig. 5l is a diagram showing MCF7 xenograft growth in mice treated w/ or w/o FULV, FULV plus weekly FMD, or combination of FULV, weekly FMD and i.p. insulin, IGF1, leptin, or the three FRFs combined (n=6/9). At day 35 (crossover), FRFs administration was withdrawn, while it was started in mice that had only received FULV plus FMD. All animal experiments were performed in six-to-eight week-old female BALB/c athymic mice (nu+/nu+).
Fig. 6a includes two histograms showing normalized protein band intensities for the PI3K/AKT/mTOR signaling cascade in ZR-75-1 and T47D cells.
Fig. 6b is an immunoblotting showing 4EBP1 and AMPK phosphorylation in MCF7 treated with TMX, FULV, STS, or their combination.
Fig. 6c includes two diagrams showing *PTEN* and *EGR1* expression level in MCF7-xenograft from six-to-eight week-old female BALB/c athymic mice (nu+/nu+) treated with FULV, TMX, FMD or their combinations; mice from all treatment groups were sacrificed and tumour masses were isolated at the end of the last FMD cycle.
Fig. 6d includes (left side) 10 photos and (right side) two histograms showing OPP-based determination of protein synthesis in MCF7, T47D and ZR-75-1 treated with TMX/FULV, STS and their combinations.
Fig. 6e is an immunoblotting showing EGR1, PTEN, GAPDH levels, and AKT phosphorylation in MCF7 cells in response to EGR1 silencing (with EGR1-shRNA#2), TMX, FULV, STS, or their combinations.
Fig. 6f includes two diagrams showing the viability of control and of EGR1-silenced MCF7 (EGR1-shRNA#2) in response to TMX, FULV, STS, or their combinations.
Fig. 6g includes an immunoblotting and a histogram showing MCF7 cell viability in response to PTEN silencing, TMX, FULV, STS, or their combination; in the upper inset, PTEN and vinculin levels in control and in PTEN-shRNA-expressing MCF7 were detected by Western blotting.
Fig. 6h is a histogram showing *EGR1* and *PTEN* expression detected in control and in myr-AKT-overexpressing MCF7 cells treated w/ or w/o TMX/FULV plus STS.
Fig. 7 includes four immunoblottings showing that AMPK phosphorylation regulation in response to ET and STS is dependent on AKT inhibition. AMPK phosphorylation and vinculin levels in MCF7 with silenced PTEN or expressing myr-AKT and treated w/ or w/o TMX/FULV, STS and their combinations were detected by Western blotting.
Figs. 8a-b show Gene Set Enrichment analyses of MCF7 treated with TMX, ST8eS and combination.
Figs. 8c-d are histograms showing *E2F1*/*E2F2*/*CCND1*/*CCNE1* mRNA expression and quantification of phosphorylated RB protein in T47D and ZR-75-1 cells treated with TMX/FULV, STS and their combinations.
Figs. 8e-f are diagrams showing Cell cycle analyses of MCF7, T47D, or ZR-75-1 cell treated w/ or w/o TMX/FULV and STS.
Fig. 8g is a histogram showing doubling time of control MCF7 and of ex vivo-cultured MCF7 that were resistant to FULV plus PALB (RES-MCF7) upon treatment with FULV, STS, PALB or their combinations.
Fig. 8h shows Hematoxylin and eosin (HE) staining and immunohistochemical staining for Ki67 of xenografts of control MCF7 or RES-MCF7 (established in six-to-eight week old female NOD/SCID mice) upon treatment with FULV plus PALB for 35 days (n=6 mice/group).
Fig. 9a includes seven diagrams showing Serum leptin, adiponectin, c-peptide, IGF1, IGFBP1, IFGBP3 and glucose before and after a FMD cycle in BC patients treated with ET and FMD in the NCT03340935 trial.
Fig. 9b includes two histograms showing IGF1 and c-peptide levels in serum from mice treated with FULV, TMX, weekly FMD or their combination for two weeks, one week after the end of the last FMD cycle.
Fig. 9c is a diagram showing survival of MCF7 xenograft-bearing mice after a one-month treatment w/ or w/o TMX, FULV, weekly 48h FMD, or their combinations, followed by observation.
Fig. 9d includes two diagrams showing MCF7 xenograft growth after a one-month treatment w/ or w/o TMX, FULV, weekly 48h fasting, or their combinations, followed by observation, and corresponding mouse survival.

Table 1 illustrates results from clinical trials NCT03595540 (pt. #1-24) and NCT03340935 (pt.#25-36).

Table 2 shows the complete blood counts and metabolic panel post FMD in certain patients of the NCT03595540 clinical trial.

Table 3 is a list of primers.

### Detailed description

Growth factor signaling through the PI3K/AKT/mammalian target of rapamycin (mTOR) and MAP kinase axes enhances ER activity and is a key mechanism underlying endocrine resistance^{1,3,7}. Water-only fasting (fasting) or plant-based, low-calorie, carbohydrate- and protein-restricted FMDs reduce circulating growth factors, such as insulin and IGF1^{13,7,8}. Therefore, the Applicants hypothesized that these dietary interventions could be used to enhance the activity of endocrine therapy (ET) and delay endocrine resistance.

In WO 2017/140641, the present Applicants disclosed that low-serum and low-glucose cell culture conditions designed to mimic the effects of fasting/FMD (STS) increased the antitumor activity of tamoxifen (TMX) or fulvestrant (FULV) in HR+/HER2- BC cell lines, and similar results were obtained in mouse xenografts of the same cell lines with weekly cycles of fasting or FMD.

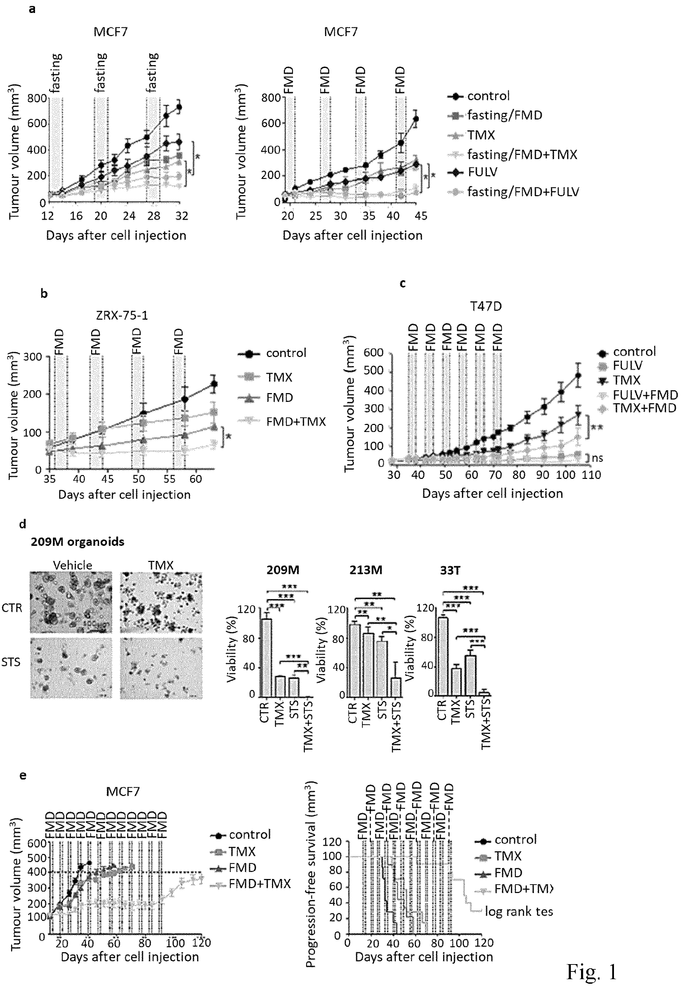

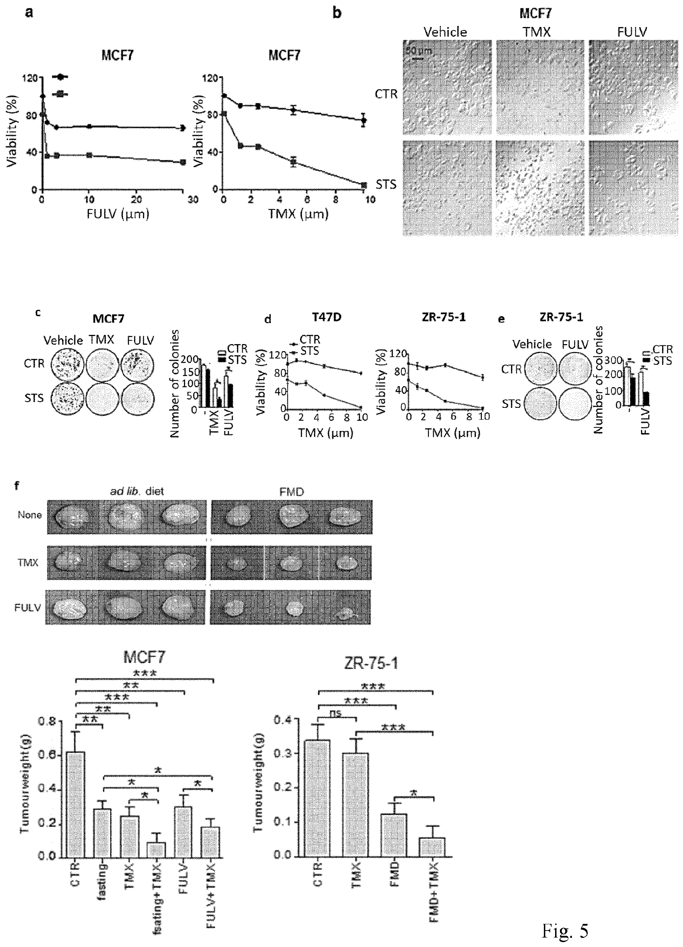

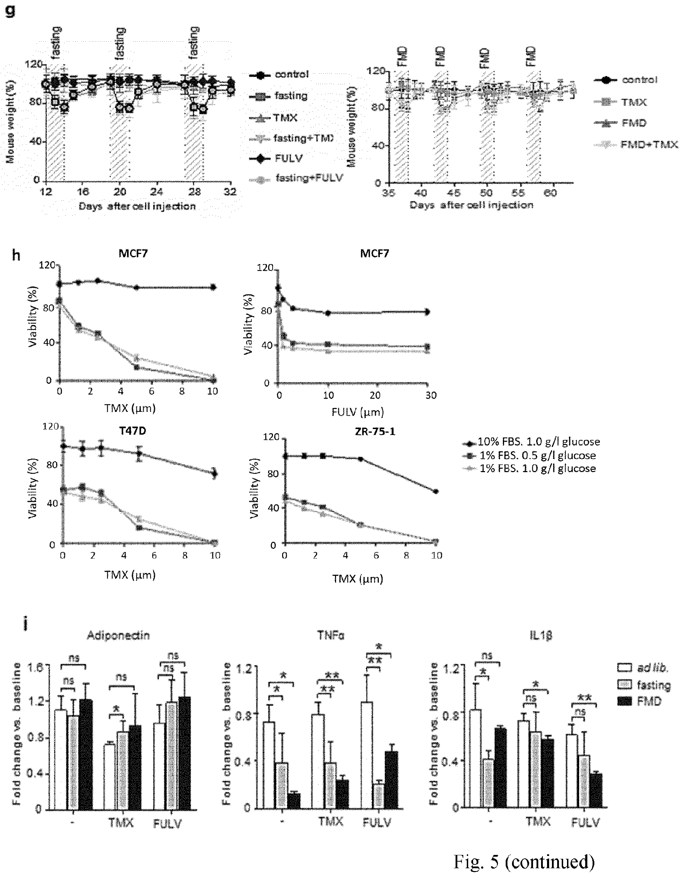

The enhancement of ET activity through STS was dependent on the reduction in serum, but not in glucose, since adding back the latter to cell growth media did not affect the observed potentiation (Fig. 5h). STS increased the antitumor activity of TMX in metastases-derived organoids from patients with HR+ BC⁹ and weekly cycles of FMD prevented acquired resistance to TMX in mice (Fig.1d-e).

In addition to increasing β-hydroxybutyrate levels (Fig.1f) and to lowering blood glucose (from 6.3±0.6 mmol/l to 4.1±0.3 mmol/l and 4.0±0.9 mmol/l with fasting and FMD, respectively; n=4), fasting and FMD w/ or w/o ET significantly modified circulating growth factors levels (Fig.1g). Serum c-peptide (a proxy of endogenous insulin production) was blunted by both dietary interventions. Fasting and FMD reduced both circulating IGF1 and IGFBP3 (which binds more than 10 80% of circulating IGF1 and protects it from rapid degradation¹⁰), while increasing IGFBP1 (which inhibits IGF1 action by binding to IGF1 itself and preventing its binding to IGF receptors).

Thus, the combined effect of these diets was a marked reduction in IGF1 levels and bioavailability. The levels of leptin, an adipokine that acts as a growth factor for HR+ BC cells and reduces ET efficacy¹¹⁻¹³, were only reduced when fasting or FMD were combined with ET. Adiponectin, which exerts antitumor effects, was only affected by the combination of fasting and TMX (Fig. 1i).

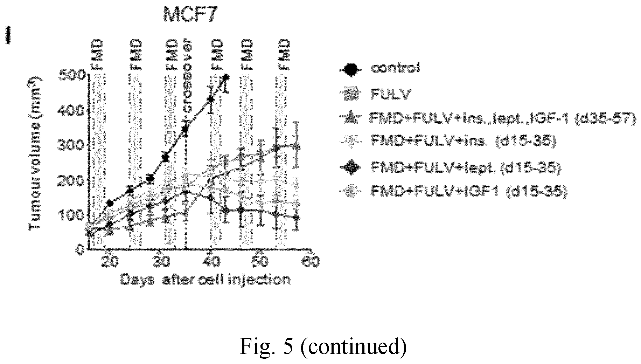

To define the role of insulin, leptin, and IGF1 in the FMD-induced potentiation of ET antitumor effects, the Applicants administered each of these factors, which are hereby collectively referred to as Fasting-Reduced Factors (FRFs), to MCF7 xenograft-bearing mice that were being treated with FULV plus FMD (Fig.1h, Fig. 5l).

Adding back any of these factors (or their combination) was sufficient to revert the FMD-induced enhancement of FULV activity (Fig. 1h).

Withdrawing the FRFs restored tumor sensitivity to ET plus FMD, while administering them to mice that had only received FULV plus FMD stimulated tumor growth and abrogated the FMD-induced potentiation of FULV (Fig. 1h, after crossover).

These data are consistent with a previous study demonstrating the key role of insulin reduction in the enhancement of the efficacy of PI3K inhibitors in various cancer types¹⁴, but show that the concomitant reduction of additional factors with growth-promoting ability is also important for this therapeutic effect to be achieved. The fact that all these FRFs have overlapping effects in the activation of signaling cascades regulating HR+ BC sensitivity to ET, and in particular the PI3K-AKT-mTOR pathway^{11,12,14-17}, may explain why reducing all three is required to promote ET antitumor activity.

Consistent with this notion, tumors isolated from mice from which FRFs were withdrawn exhibited reduced phosphorylation of AKT and of p70S6K (a mTOR target), while in tumors from animals administered FRFs during treatment with FULV plus FMD, we found levels of AKT and p70S6K phosphorylation that were comparable to those of *ad lib.* fed mice (Fig. 1i).

The Applicants monitored the effect of fasting/FMD on additional circulating factors with a reported role in BC pathophysiology¹⁷, and found that both fasting and FMD (with or without TMX/FULV) downregulated TNFα, which acts pro-oncogenically by upregulating aromatase in the tumor microenvironment and by enhancing angiogenesis and cell invasion, and IL1β, which enhances HR+ BC cell migration and metastasis^{17,18} (Fig.1i).

Since insulin, IGF1, and leptin down-regulation proved essential for FMD-induced potentiation of ET antitumor activity, the Applicants focused on these FRFs for subsequent mechanistic experiments. However, it is likely that the benefit of fasting/FMD to ET may involve other mediators, such as TNFα and IL1β.

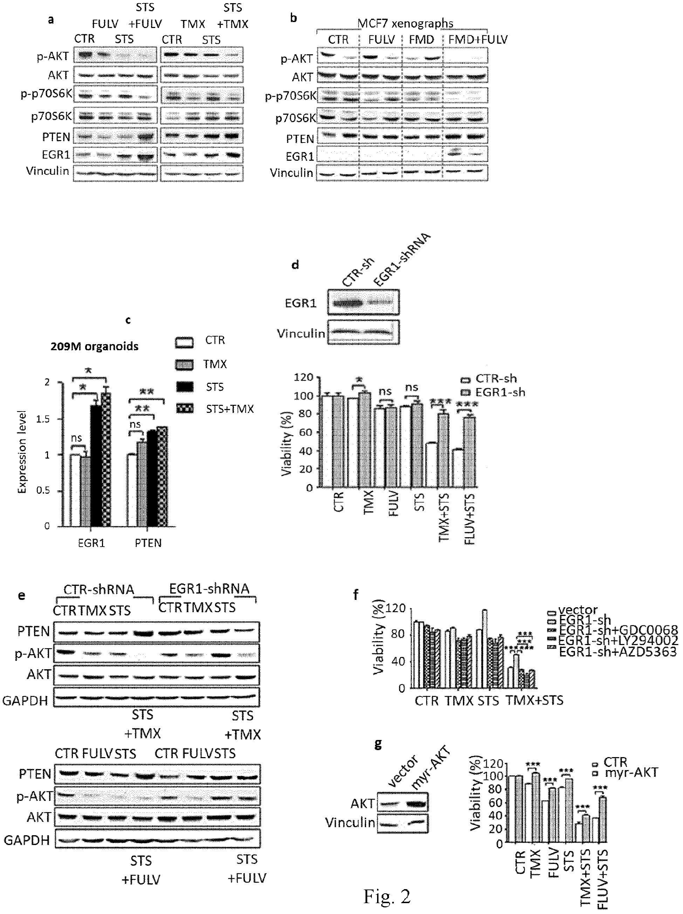

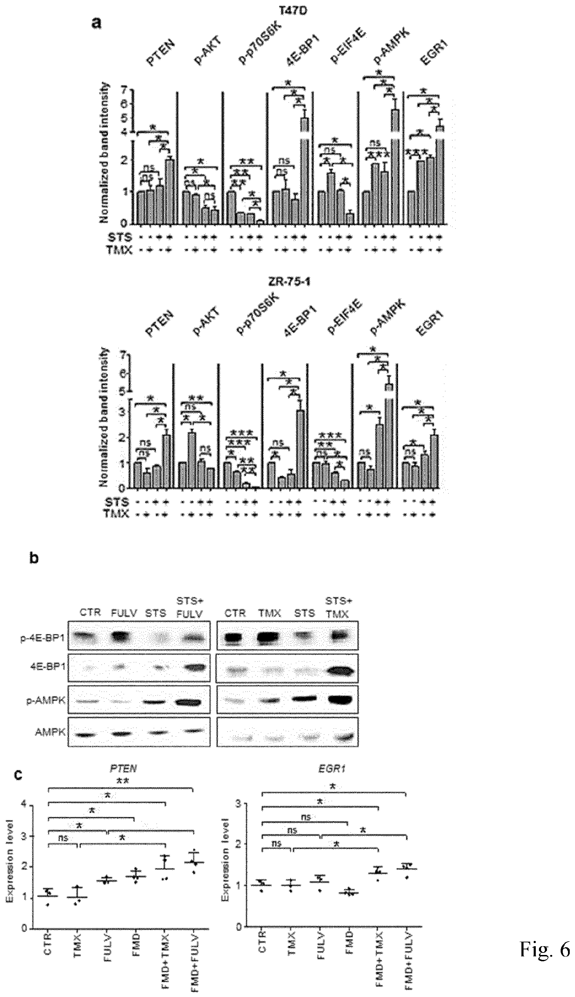

STS and the FMD (in experiments with MCF7 xenografts) cooperated with ET to increase the expression of PTEN, an upstream negative regulator of AKT-mTOR signaling, in HR+ BC cell lines and in HR+/HER2- BC organoids (Fig.2a-c, Fig. 6a). In line with the previously described reduction of AKT phosphorylation and mTOR activity (Fig.1i), these treatment combinations reduced p70S6K and eIF4E phosphorylation, increased the translational repressor 4E-BP1, primarily in its unphosphorylated form, and inhibited protein synthesis (Fig. 2a,b and Fig. 6a-d).

Among the known enhancers of PTEN expression, the Applicants focused on the tumor suppressor EGR1 (whose expression is associated with good prognosis in BC patients^{19,20}) since they previously showed that EGR1 is upregulated in healthy tissues during fasting²¹ and because EGR1 is suppressed by ER activity²².

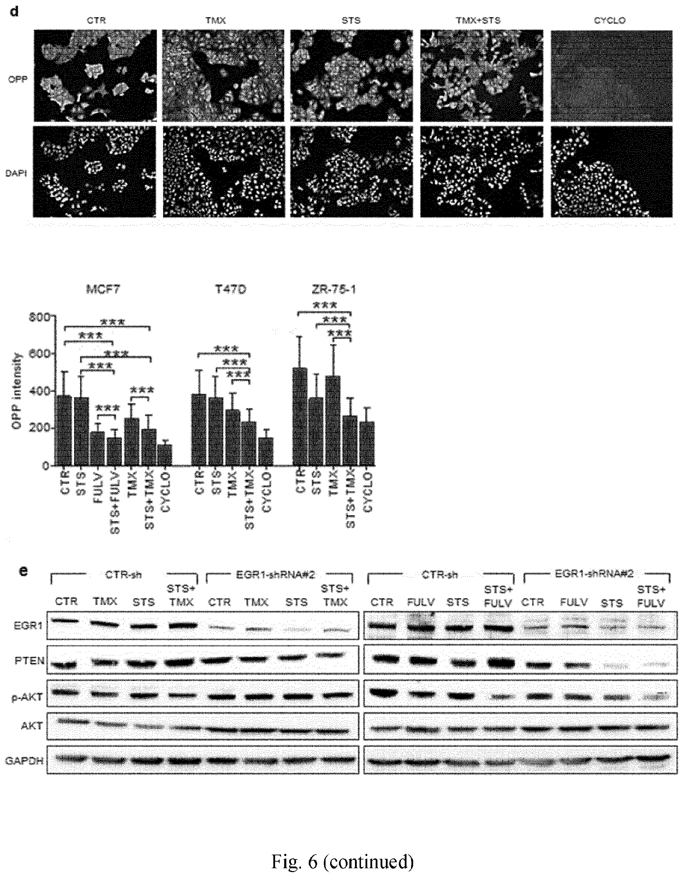

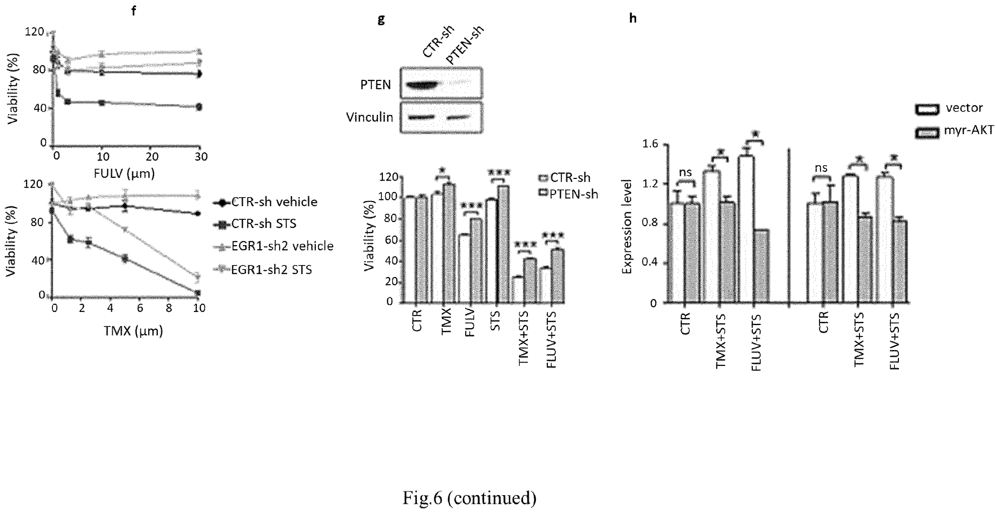

EGR1 was increased upon treatment of HR+ BC cell lines and HR+ BC organoids with ET plus STS (Fig.2a-c and Fig. 6a,b). EGR1 silencing reduced the antitumor activity of the ET-STS combination and prevented PTEN accumulation, as well as the reduction of AKT phosphorylation in response to this combination (Fig. 2d,e and Fig. 6e,f). BC cell protection from combined ET and STS through EGR1 silencing reflected persistent AKT activity, since the AKT inhibitors, GDC0068 and AZD5363, and the PI3K inhibitor, LY294002, all abolished such protection (Fig.2f).

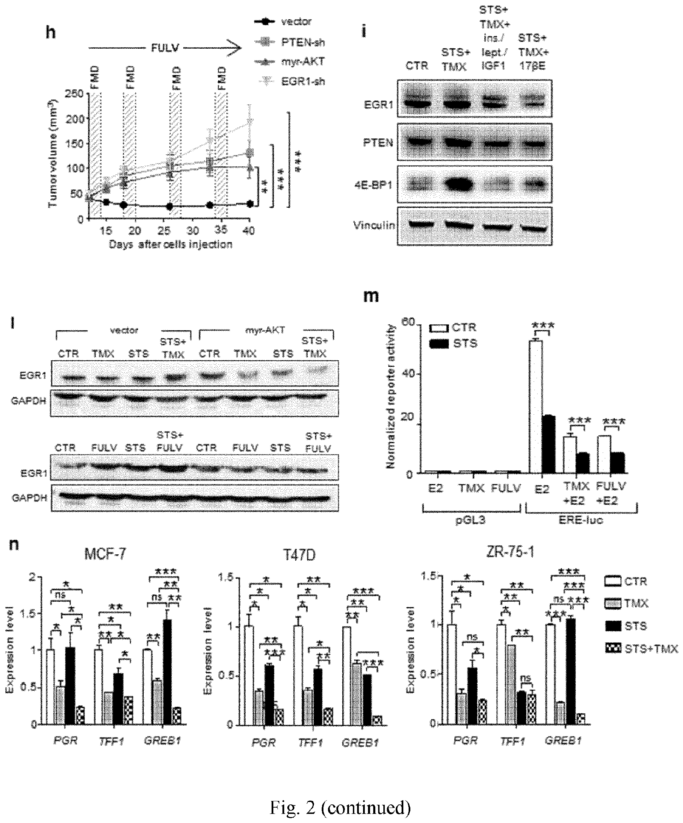

PTEN silencing and expression of a constitutively active AKT [myristoylated (myr)-AKT] also reduced MCF7 sensitivity to combined ET and STS/FMD (Fig.2g,h; and Fig. 6g). Supplementation with insulin, IGF1 and leptin, or with 17β-estradiol prevented the increase in EGR1, PTEN and 4E-PBP1 that was induced by the TMX-STS combination in MCF7 cells (Fig.2i).

In addition, EGR1 and PTEN upregulation were also abolished by myr-AKT (Fig.2l and Fig. 6h). Therefore, fasting/FMD and ET appear to cooperate to reduce AKT-mediated inhibition of EGR1 expression. In turn, increased EGR1 leads to higher PTEN levels and strengthens AKT inhibition itself. AMP-activated kinase 17 (AMPK), which negatively controls mTOR activity, was also phosphorylated on an activating residue in BC cells that were exposed to combined ET and STS (Fig. 6a,b).

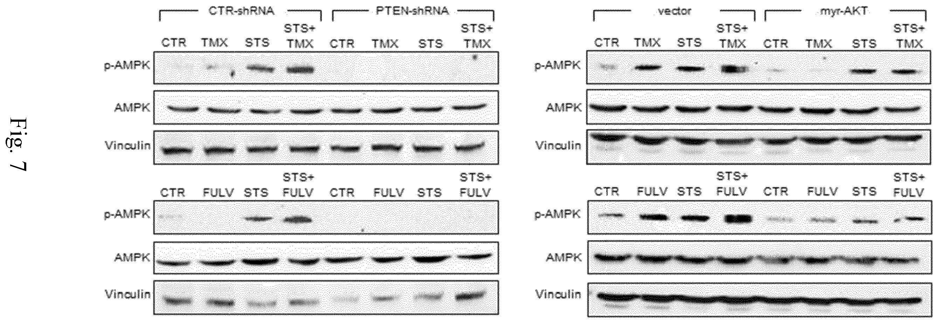

This effect was prevented by both PTEN silencing and myr-AKT (Fig. 7), thus indicating that AMPK stimulation in response to coupled ET-STS is secondary to PTEN upregulation and to reduced AKT activity²³.

ER activity is essential for HR+ BC cell survival and proliferation, and is enhanced by insulin, IGF1 and leptin signaling at multiple levels^{1,8,11,16,24}. STS reduced ER transcriptional activity and enhanced FULV- and TMX-mediated ER inhibition in HR+ BC cell lines, as indicated by luciferase reporter assays and by the reduction in the expression of the ER target genes *TFF1, PGR,* and *GREBI* (Fig.2m,n). Thus, STS and ET also cooperate to inhibit ER activity.

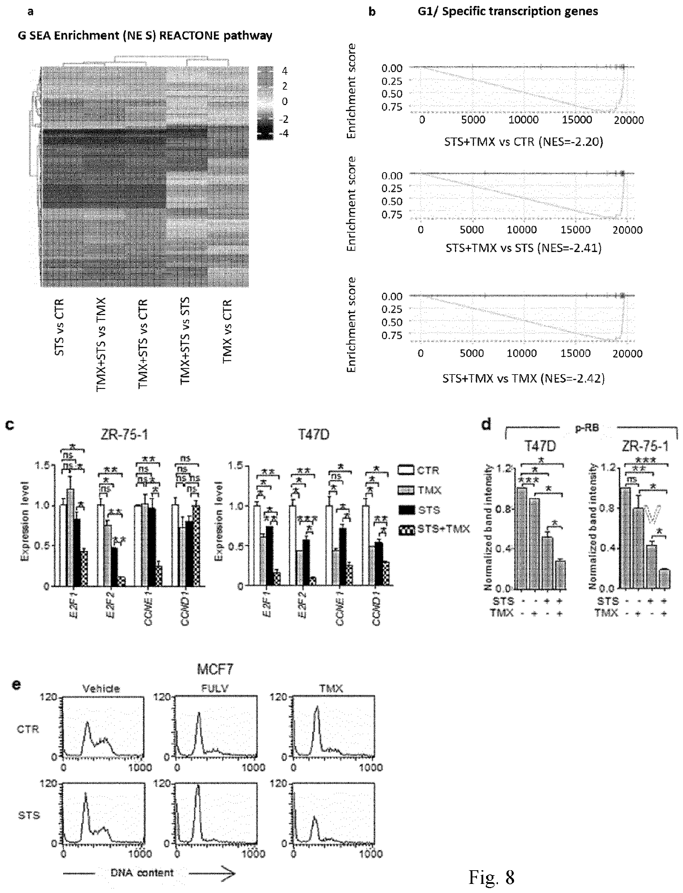

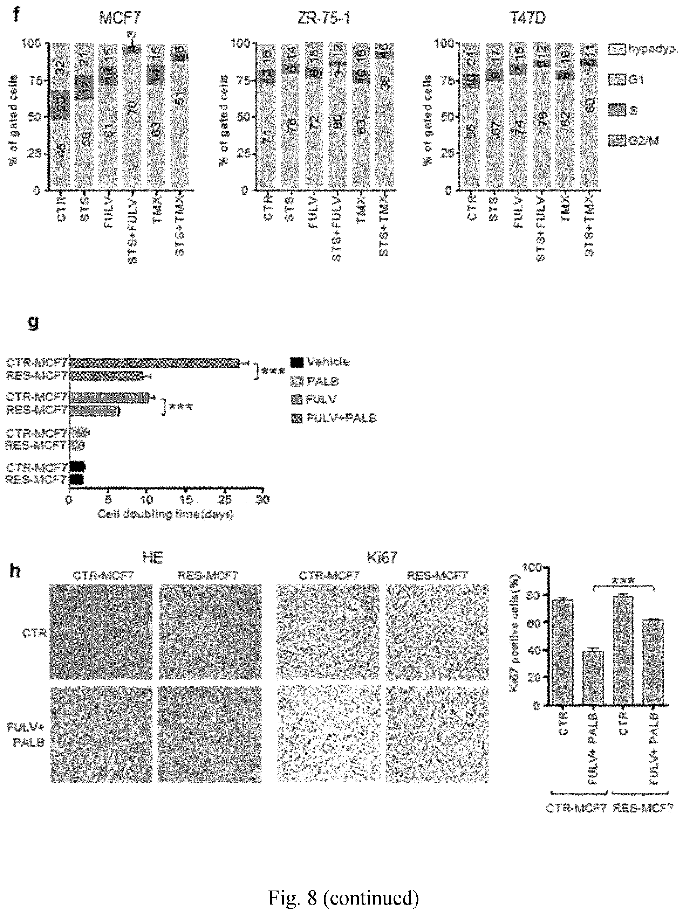

Using gene expression microarrays and Gene Set Enrichment Analysis (GSEA), the Applicants found a downregulation of gene categories belonging to cell cycle-related themes through combined TMX and STS in MCF7 cells (Fig.3a and Fig. 8a,b). E2F1, E2F2, CCNE1 and 5 CCND1 downregulation was verified after combined treatment at the mRNA (Fig.3b and Fig. 8c) and at the protein level (CCND1; Figure 3c,d).

Consistent with their effects on cell cycle regulators, STS and ET together reduced phosphorylated RB in HR+ BC cells and induced cell cycle arrest in the G0-G1 phase (Fig.3c and Fig. 8d-f). EGR1 silencing and myr-AKT expression both attenuated CCND1 downregulation in response to coupled ET-STS, and myr-AKT also protected MCF7 from the cell cycle arrest imposed by ET, STS and their combination (Fig.3e,f). Therefore, EGR1 upregulation and AKT inhibition act upstream to mediate CCND1 downregulation through combined ET-STS and the consequent cell cycle arrest. 13

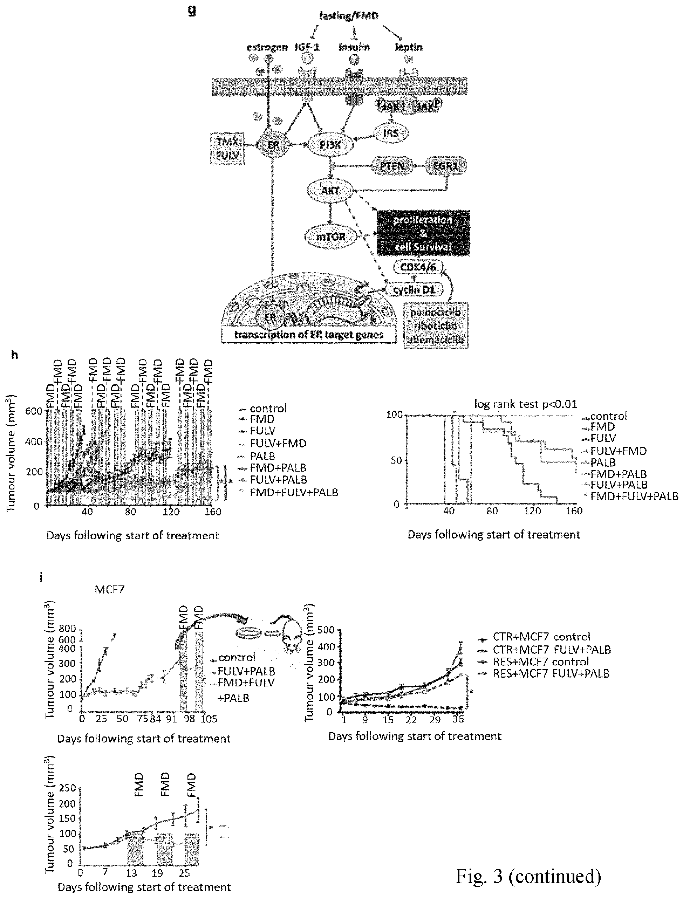

Thereafter, the Applicants reasoned that CCND1 downregulation via the combination of ET and STS could be exploited to provide an additional therapeutic effect, by adding a CDK4/6 inhibitor, such as palbociclib (PALB; Fig.3g). In orthotopic MCF7 xenografts-bearing mice, FMD cycles and PALB postponed the occurrence of resistance to FULV to a similar extent (Fig.3h).

However, even with these combinations, resistance acquisition and tumor progression could not be avoided. In contrast, combining FULV, FMD and PALB not only prevented tumor growth for over 160 days, but also led to a slow, but steady, tumor shrinkage. Furthermore, administering FMD cycles to mice with tumors that had already become resistant to FULV plus PALB induced tumor shrinkage even at this advanced stage (Fig. 3i).

The ability of MCF7 cells from resistant tumors to grow in the presence of FULV and PALB was verified *in vitro* and *in vivo* (Fig.3i and Fig. 8g,h). Resistant MCF7 cells were used to establish new xenografts, and, again, adding the FMD to FULV and PALB resulted in antitumor activity against these re-transplants (Fig.3i, right panel).

The combination of periodic FMD and ET was tested in 36 patients with HR+ BC, who were enrolled in the NCT03595540 (pt.#1-24) and in the NCT03340935 (pt.#25-36) clinical trials.

In the former trial, patients received a previously reported 5-day FMD, every four weeks. They completed on average 6.1 FMD cycles, with some of them receiving up to 13 cycles. Also in this clinical study, the FMD proved to be safe, only leading to G1-2 adverse events, most commonly headache (41%) and fatigue (21%) (Table 1 and 2). Patients from the NCT03340935 21 study received a similar, albeit more calorie-restricted, FMD regimen every 3-4 weeks and completed an average of 5.5 cycles with no severe adverse events (Table 1).

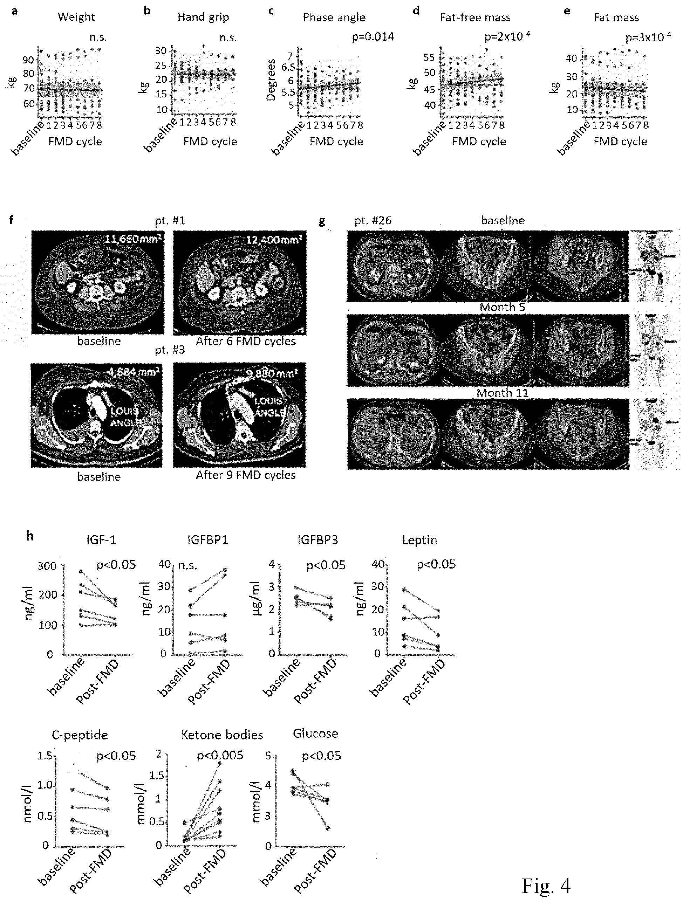

Clinical outcomes in patients with metastatic HR+/HER2- BC, including those treated with combined ET, PALB and FMD (n=4), are promising (Fig.4g and Table 2). Pt.#1, #26 and #27 have been treated in the second-line treatment setting for 30, 18 and 11 months, respectively, receiving a total of 10 (Pt.#1) and 8 (Pt.#26, #27) FMD cycles. P.#1 and Pt.#26 still have clinically controlled disease, while Pt.#27 progressed after 11 months (median progression-free survival in this clinical setting is 9 months^{15,27,18,28}. Pt.#29 received fourth-line treatment with FULV and PALB plus 5 FMD cycles, ultimately progressing after 11 months.

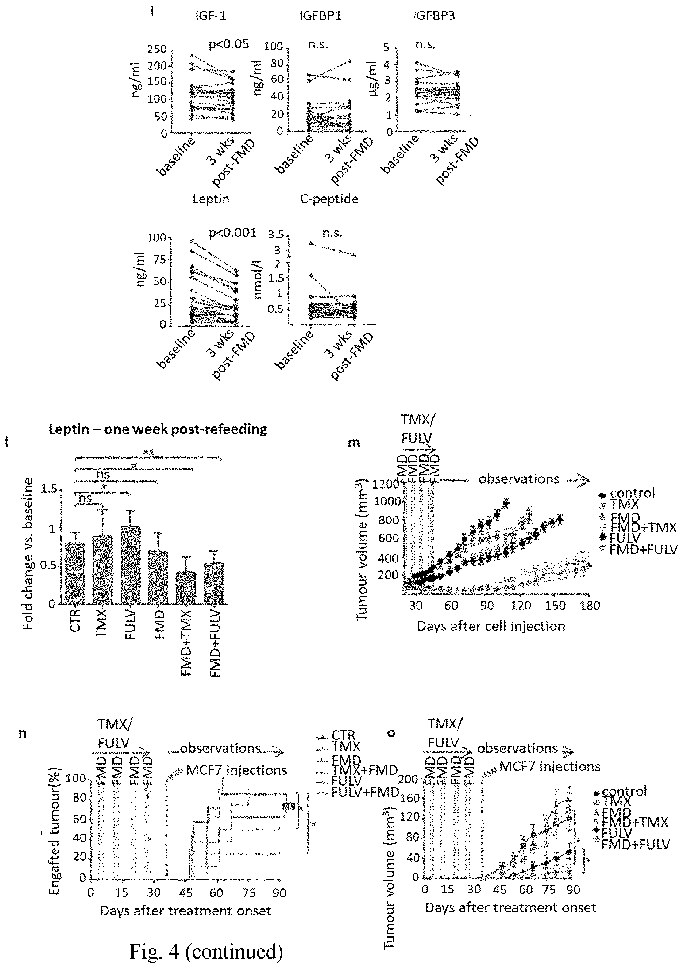

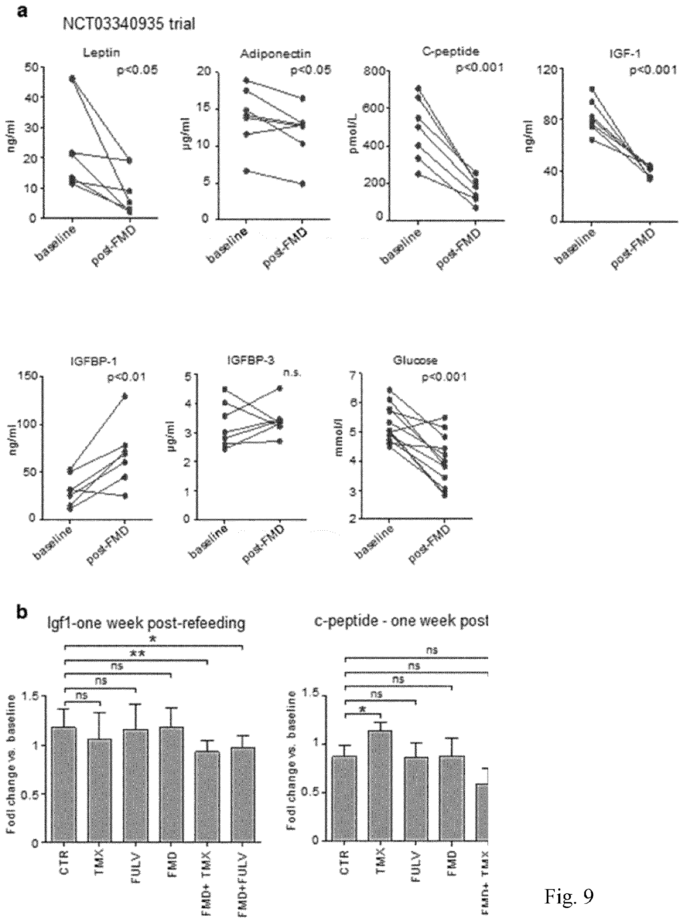

Considering all patients with HR+/HER2- BC enrolled in these trials, the FMD lowered blood glucose, serum IGF1, leptin, and c-peptide, while increasing circulating ketone bodies (Fig.4h and Fig.9a). Leptin and IGF1 (but not c-peptide) levels were still lower than the baseline values three weeks after the end of the FMD (Fig.4i).

Similar findings were obtained in mice (Fig.4l and Fig.9b), where, in response to the ET-FMD combination (but not to ET or FMD alone), leptin and IGF1 (but not c-6 peptide) remained lower despite refeeding one week after the end of the FMD as compared to ad lib. fed mice.

Since both leptin and IGF1 stimulate BC cell proliferation^{11,16,24,29,30}, it was evaluated whether these changes in circulating FRFs, which persisted beyond the FMD period, were associated with anticancer effects.

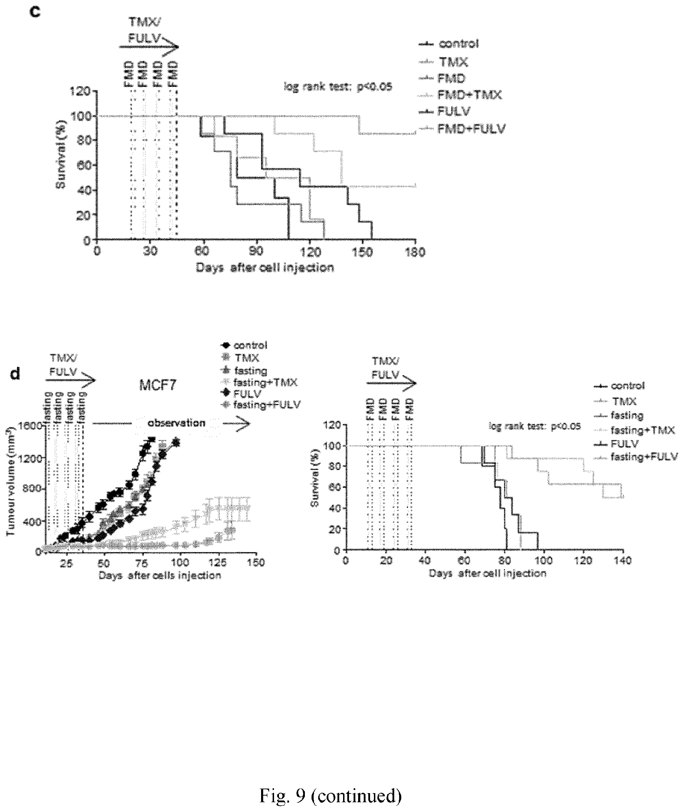

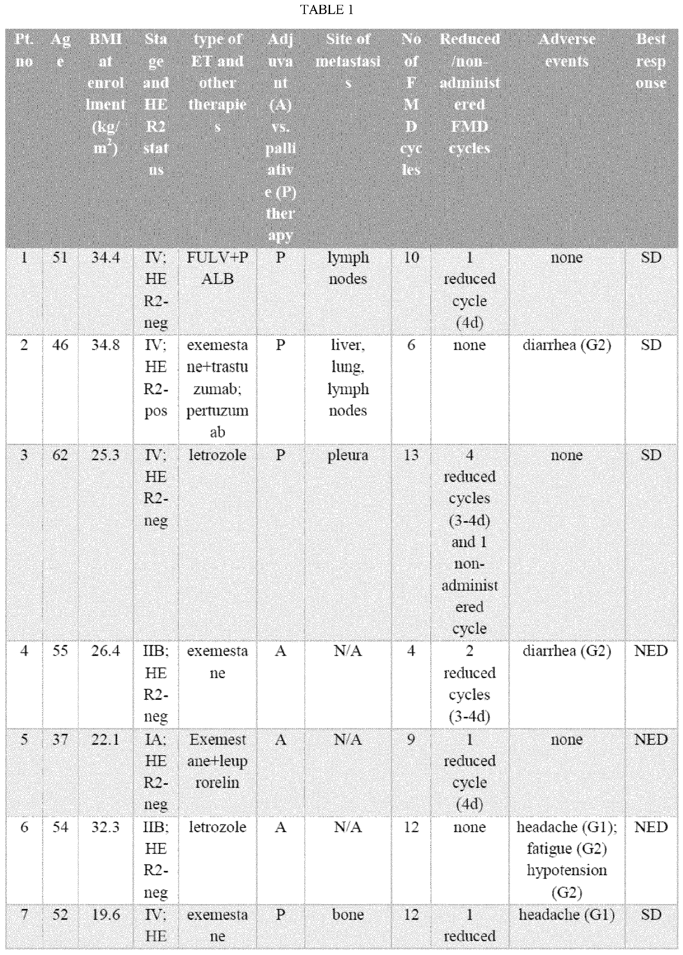

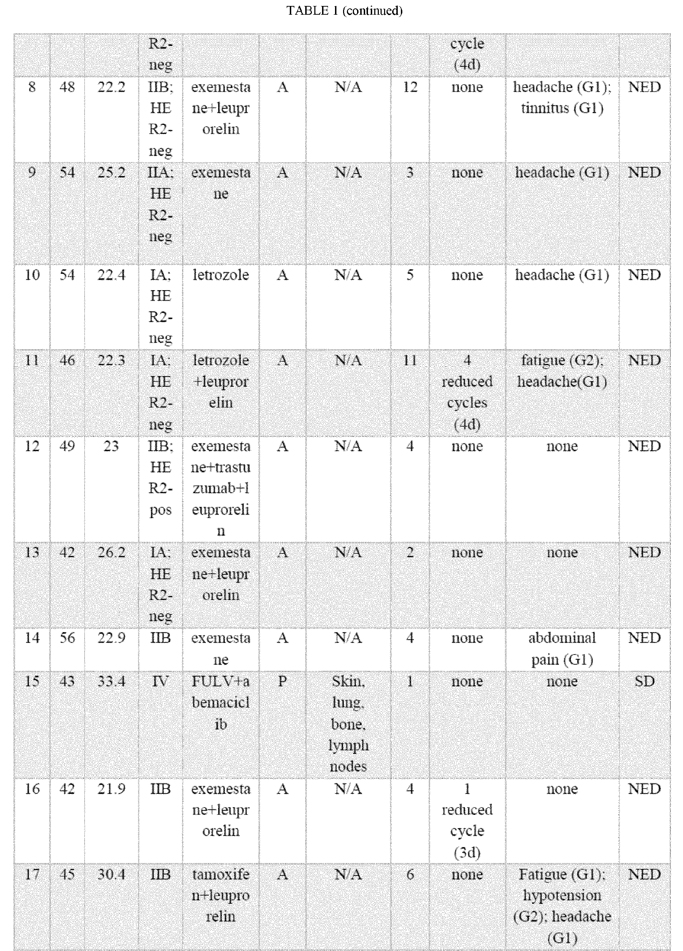

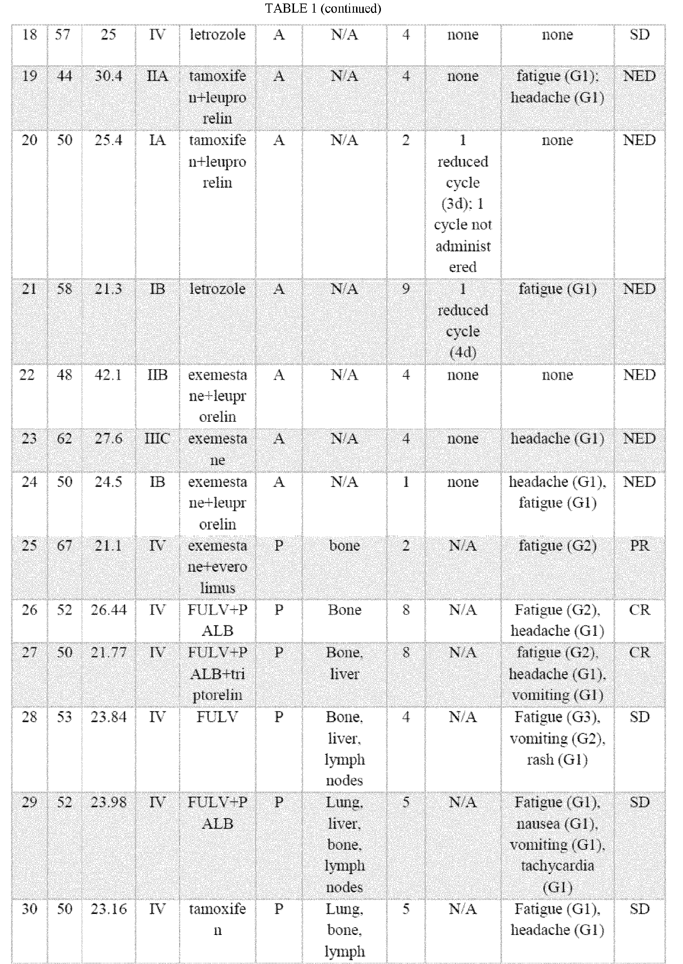

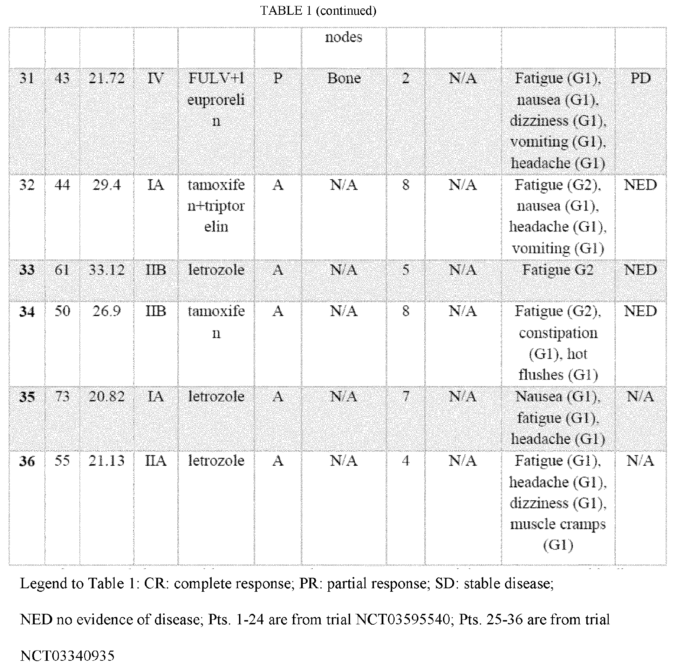

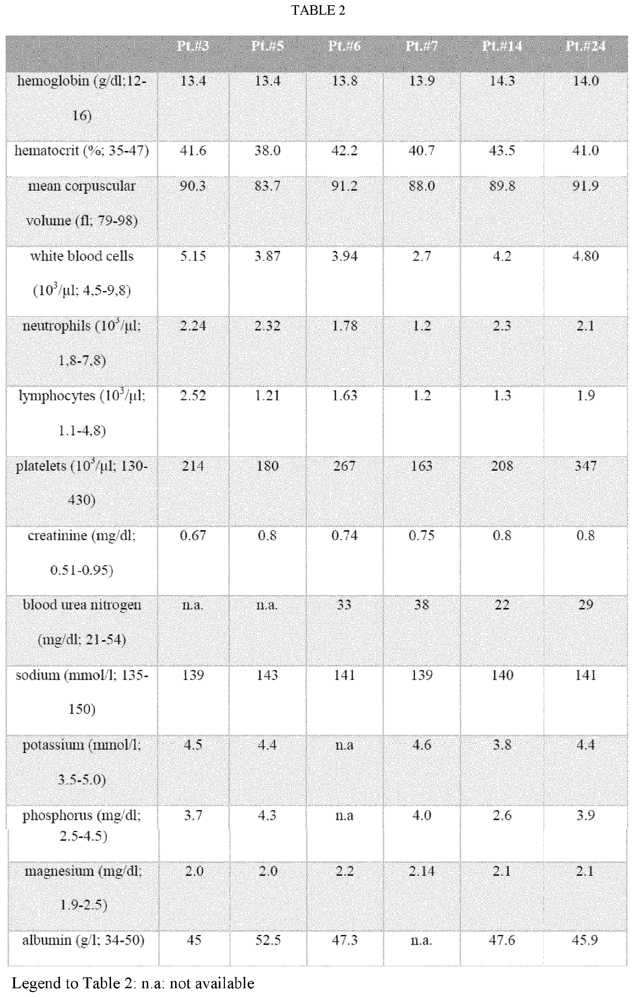

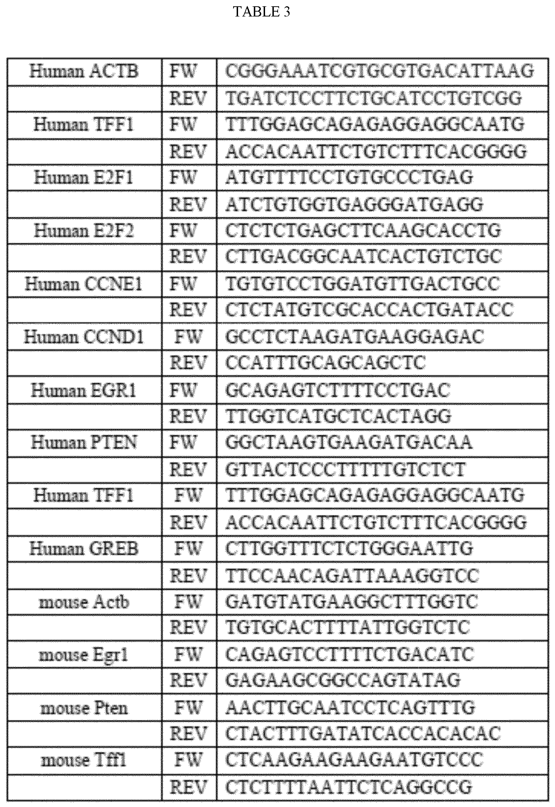

A one-month treatment of MCF7 xenograft-bearing mice with ET plus FMD (or fasting), but not with the single treatments, slowed tumor growth for up to 90 days after treatment withdrawal, and also resulted in enhanced mouse survival (Fig.4m and Figs. 9c,d).

In addition, mouse pre-treatment with coupled ET and FMD for one 13 month, followed by MCF7 cell inoculation, reduced tumor engraftment and slowed the growth of those tumors that did engraft (Fig.4n,o). Pre-treatment with FULV also slowed MCF7 xenograft growth, likely due to the use of a long-acting formulation of this agent in our study. However, pre-treatment with combined FMD and FULV was more active than FULV alone.

In conclusion, periodic cycles of fasting/FMD increase the anti-cancer activity of TMX and FULV, delay resistance to these agents and, in combination with FULV and PALB, cause tumor regression and reverse acquired resistance to these two drugs.

Pivotal for fasting/FMD-induced enhancement of ET antitumor activity appears to be the simultaneous reduction in blood IGF1, insulin and leptin levels, with the consequent inhibition of the PI3K-AKT-mTOR signalling pathway, at least in part via EGR1 and PTEN upregulation (Fig.3g).

Of note, fasting/FMD provide AKT-mTOR axis inhibition without causing the rebound hyperglycemia and hyperinsulinemia that are instead associated with the use of pharmacologic inhibitors of PI3K or mTORC1, and which were implicated in tumor resistance to these treatment ^{14,31,32}.

The leptin- and IGF1-lowering effect of ET plus FMD persists beyond the FMD period and is associated with carryover anticancer activity. Therefore, the observed regression of HR+ BCs in mice could result from the combination of an acute and stronger reduction of FRFs, together with milder, chronic reduction of the same FRFs. In fact, the long-term reduction in leptin and IGF1 may contribute to generate an extended inhospitable environment for HR+ BC cells.

### Methods

### Cell Lines and Reagents

MCF7, ZR-75-1 and T47D cell lines were purchased from ATCC (LGC Standards S.r.l., Milan, Italy). Cells were passaged for less than 6 months before resuscitation for this study. Recombinant human IGF1 and recombinant human leptin were purchased from Peprotech. Insulin (Humulin R) was obtained from the Pharmacy of the IRCCS Ospedale Policlinico San Martino. Puromycin, protease/phosphatase inhibitor cocktail, β-estradiol, sulforhodamine B, TMX and FULV (for in vitro use) were purchased from Sigma Aldrich S.r.l. Fulvestrant for in vivo use was purchased from Astrazeneca (Faslodex). PALB for in vitro experiments was purchased from Selleck Chemicals while for in vivo experiments it was purchased from Medchem Express. 17β-estradiol-releasing pellets were purchased from Innovative Research of America.

### Cell viability assays and cell doubling time estimation

2.8×10³ MCF7, 5×10³ T47D or 5×10³ ZR-75-1 cells were plated in 96 well plates in CTR medium. After 24h medium was removed, cells were washed with PBS and incubated either in CTR (10% FCS and 1g/L glucose) or STS (1% FBS, 0.5g/L glucose) medium. After 24h cells were stimulated w/ or w/o with TMX or FULV at the indicated concentrations. Where indicated, GDC0068, AZD5363 and LY294002 (from SelleckChem) were used at 1µM, 400 nM and 2µM concentration, respectively. Viability was determined 72h later by CellTiter96 Aqueous1 (Promega) according to the manufacturer's instructions.

### Organoid culture and viability assays

Organoid lines used were previously published by Sachs et al. and were cultured and passaged as previously described. For viability assays organoids were collected, washed, filtered with a 70 µm nylon filter (Falcon)and brought to a 7.5×10³ organoids/mL density in regular medium or STS 25 medium (0.5 g/L for STS and concentration of B27 brought to 0.1x instead of 1x) containing 5% BME. Thereafter, 40 µL organoid suspension was dispensed in each well of a 384-well plate (Corning) using a Multi-drop Combi Reagent Dispenser (Thermo Scientific). Organoids were subsequently treated withTMX (0.01-55 µM) with 4 replicates for each concentration. TMX was dispensed using the Tecan d300e digital dispenser. DMSO concentrations never exceeded 1%. Five days later, viability was assessed using CellTiterGlow 3D reagent (Promega) according to the manufaturer's instructions. Cell viability was normalized based on the signal obtained in organoids treated with 1 pM staurosporin (Sigma Aldrich; positive control; corresponding to 0% cell viability) and DMSO (negative control, corresponding to 100% viability).

### Retroviral and lentiviral transduction

pBABE-puro (PBP), PBP-myr-AKT, pMKO-GFP-shRNA, pMKO-PTEN-shRNA the and lentiviral packaging plasmid (pCMV-dR8.2 dvpr and pCMV-VSV-G) were purchased from Addgene (Cambridge, MA, USA).

pLKO and pLKO-EGR1-shRNA1-3 were purchased from Sigma Aldrich S.r.l. Retro- and lentiviral transdution was performed as described elsenwhere³⁴.

### Immunoblotting

For protein lysate generation from cultured cells, 5×10⁴ MCF7, T47D or ZR-75-1 cells were plated in 6-well plates in CTR medium. After 24h medium was removed, cells were washed with PBS and incubated either in CTR or STS medium. After 24h cells were stimulated w/ or w/o with TMX (5 pM) or FULV (10 pM). Where indicated, cells were supplemented with insulin (400 pM), IGF1 (5 ng/ml), leptin (50 mg/ml), or 17β-estradiol (100 nM). 24h later, cells were washed and protein lysates were generated as described elsewhere^{34,35}. Protein lysates from primary tumors or mouse uteri were obtained using TissueRuptor (Quiagen). Proteins (35pg) were separated by SDS-PAGE, transferred to a PVDF membrane (Immobilon-P, Millipore S.p.A.), and detected with the following antibodies: anti-phospho-AKT (Ser473; #4058), anti-AKT (#9272), anti-PTEN (#9552), anti-phospho-p70S6kinase (Thr389; #9206), anti-p70S6kinase (#9202), anti-4E-BP1 (#4923), anti-phospho-eIF4E (Ser209; #9741), anti-phospho-RB (Ser807/811; #9308), anti-RB (#9309), anti-CCND1 (#2978) were all from Cell Signaling Technology; anti-phospho-AMPK (Thr172; PA5-2 17831), anti-AMPK (PA5-29679), anti-EGR1 (MA5-15008) were from Thermo Fisher; anti-β-actin was from Santa Cruz Biotechnology. Band intensities were quantified by Quantity One SW software (Bio-Rad Laboratories, Inc.) using standard enhanced chemiluminescence.

### Colony formation assays

8×10² MCF7 or 2×10³ ZR-75-1 cells were plated in 6-well plates in regular medium. 24h later cell medium was removed. Cells were washed twice with PBS and were incubated either in CTR or STS medium. The next day cells were treated with either vehicle DMSO, 5 pM TMX or 30 pM fulvestrant for 24h. Then cell medium was removed and cells were cultured for two weeks. Cell colonies were finally enumerated as described in³⁴.

### Cell cycle analysis by flow cytometry

Cell cycle analysis of cultured BC cells was performed as described elsewhere³⁵.

### Protein Synthesis Assay

7.5×10³ MCF7, 1×10⁴ T47D or ZR-75-1 cells were plated in 96-well plates in CTR medium. After 24h the cell medium was removed, cells were washed with PBS and then incubated either in CTR or STS medium. 24h later cells were stimulated w/ or w/o TMX (5 pM) or FULV (10 pM). Protein synthesis was determined with Click-iT^{™} Plus OPP Alexa Fluor^{™} 488 Protein Synthesis Assay Kit (Life Technologies) with PerkinElmer Operetta^{®} High Content Imaging System.

### Luciferase reporter assay

For ER luciferase reporter assays, 4×10⁴ MCF7 cells were seeded in a 24-well plate 48h before transfection. Cells were grown in phenol red free regular medium, supplemented with 10% charcoal stripped FBS (Gibco). Cell medium was then replaced and substituted with either regular or STS phenol red free medium, containing 10% or 1% charcoal treated FBS respectively, and 70% confluent cells were transfected with TransIT^{®}-LT1 Transfection Reagent (Mirus). 350 ng of pGL3 promoter plasmid (Promega) or of the pS2/TFF1 reporter vector containing 1.3 kb of the proximal promoter of the estrogen-responsive gene TFF1 cloned in the pGL3-basic backbone³⁶ were used together with 150ng of the pRLSV40 plasmid (Promega), which harbours the luciferase gene from Renilla reniformis under a constitutive promoter. The next day, transfected cells were treated w/ or w/o TMX (5 µM), FULV (10 pM), 17β-estradiol (1 nM) and harvested after additional 24h. Luciferase signals were measured using the Dual-Luciferase^{®} Reporter Assay System (Promega) as 9 described previously³⁶.

### Gene expression profiles and functional analyses

For gene expression microarray studies of cultured MCF7 cells, 5×10⁴ cells were plated in 6-well plates in CTR medium. After 24h medium was removed, cells were washed with PBS and incubated either in CTR or STS medium. After 24h, cells were stimulated w/ or w/o with 5 µM TMX. 24h later, total RNA isolation was performed with the RNeasy Mini Kit (Quiagen, GmbH Hilden, Germany). RNAs were hybridized in quadruplicate on Agilent Human GE 4x44K V2 Microarray (G2519F-026652) following the manufacturer's protocol. Hybridized microarray slides were scanned with the Agilent DNA Microarray Scanner G2505C at a 5pm resolution with the manufacturer's software (Agilent ScanControl 8.1.3). The scanned TIFF images were analysed numerically and background-corrected using the Agilent Feature Extraction Software (version 21 10.7.7.1), according to the Agilent GE1_107_Sep09 standard protocol. The output of Feature Extraction was analysed with the R software environment for statistical computing (http://www.r-project.org/) and the Bioconductor packages (http://www.bioconductor.org/). The arrayQuality Metrics package was used to check the quality of the arrays. Low signal Agilent probes, identified by a repeated "not detected" flag across the majority of the arrays in every condition, were filtered out from the analysis. Signal intensities across arrays were background corrected (Edwards method) and normalized with the quantile normalization method. DEGs were determined adopting a double threshold based on 1) the magnitude of the change (fold change greater than ± 2) the statistical significance of the change, measured with multiple-test-correction adjusted p-value (q-value), less than 0.05, using Limma package. Gene set enrichment analysis was performed using the version implemented in fgsea package, performing 10000 permutations and using as database the REACTOME Pathways dataset (reactome.db package). Box-plots were generated using ggplot2 package and to evaluate the differences between the groups, non-parametric Wilcoxon-test was used.

### Quantitative real-time PCR (QPCR)

QPCR were performed as described elsewhere³⁵. Gene-specific primers were purchased from Sigma-Aldrich or Thermo Fisher and are listed in Table 3. Comparisons in gene expression were performed using the 2-ΔΔCt method.

### ELISAs

Mice whole blood was collected in Eppendorf. It was allowed to coagulate for 2h at RT, centrifuged 20 minutes at 4,000 rpm, then aliquoted into PCR tubes and stored at -80°C until subsequent use. Patients whole blood was collected in Vacuette Serum Clot Tubes, centrifuged 20 minutes at 2,100 rpm then aliquoted into small tubes and stored at -80°C until use. All the ELISAs assay to detect murine and human serum level of IGF-1, IGFBP1, IGFBP3, c-peptide, leptin and adiponectin were purchased from R&D System except for murine c-peptide which was purchased from Alpco.

### Animal models

All mouse experiments were performed in accordance with the relevant laws and institutional guidelines for animal care and use established in the Principles of Laboratory Animal Care (directive 86 /609 /EEC), upon approval by the Italian Istituto Superiore di Sanità. NOD SCID-γ mice were utilized in the experiments from Fig.3h,i. They were derived from in-house colonies at the animal facility of IFOM-IEO (Milan, Italy) and were kept for a maximum of 3 generations. Breeders (Charles River Laboratories) and experimental animals were maintained under pathogen-free conditions. Six-to-eight week old female athymic Nude-FoxN1 mice (purchased from Envigo) were utilized in the experiments from Fig.1, 2, 4 and from Figs. 6, 7, 9 at the Animal Facility of the IRCCS Ospedale Policlinico San Martino. These animals were maintained in air-filtered laminar flow cabinets with a 12-hour light cycle and food and water ad libitum. Mice were acclimatized for 1 week.

To allow MCF7, T47D and ZR-75-1 xenografts growth, the day before cell injection a 17β-estradiol-releasing pellet (Innovative Research of America) was inserted in the intra-scapular subcutaneous region under anesthesia conditions. 5×10⁶ MCF7, 8×10⁶ ZR-75-1, or 5×10⁶ T47D cells were injected s.c. to either one or both flanks of the mouse. Alternatively, 2.5×10⁶ MCF7 were injected orthotopically into the fourth abdominal fat pad (experiments from Fig.3h,i). Treatment was initiated when the tumors appeared as established palpable masses (~2 weeks after cell injection). In each experiment, mice were randomly assigned to one arms: control - ad lib. diet; treatment TMX (45 mg/kg/day in peanut oil, oral gavage³⁷⁻³⁹); FULV (150 mg/kg/once a week, s.c³⁹⁻⁴¹); 17 PALB (65 mg/kg, in H2O, oral gavage, three times a week - Mon, Tue and Fri⁴²⁻⁴⁴); IGF1 (200 ug/kg body weight, s.c., twice a day on the days of the FMD); insulin (20 mU/kg body weight s.c., on the days of the FMD); leptin (1 mg/kg body weight⁴⁶, s.c., once a day Monday through Friday, including on the days of the FMD); fasting (water only, for 48h every week^{5,47}); FMD (previously described in^{4,5}; for 48-96h every week), or combinations of these treatments as indicated. Mice were housed in a clean new cage to reduce coprophagy and residual chow. Body weight was measured immediately before, during and after fasting/FMD. FMD cycles were repeated every 5 or every 7 days in order to obtain complete recover of body weight before a new cycle. Tumor volume was calculated using the formula: tumor volume= (w2 × W) x π/6, where

"w" and "W" are "minor side" and "major side" (in mm), respectively. Tumor masses were always isolated at the end of the last FMD/fasting cycle, weighted, divided in two parts and stored in liquid nitrogen for subsequent RNA extraction, or fixed in formalin for histology. For the resistance acquisition experiments mice were treated with weekly FMD cycles, with a one-week break every fifth week, until the masses quadrupled their initial volume, as this was chosen as the criterion for defining resistance acquisition and disease progression. In Fig.3h, left panel (representing mouse progression-free survival), mice deaths, that were unrelated to disease progression, are presented as outreach symbol on the survival curves

### Clinical studies of FMDs in patients undergoing ET for HR+ BC

The NCT03595540 was conducted at the IRCCS Ospedale Policlinico San Martino. This trial consists of a single-arm phase II clinical study of a FMD (Prolon, by L-Nutra, Los Angeles, CA) in 60 patients with solid or hematologic tumors undergoing active cancer treatment. It is aimed at assessing feasibility, safety and effects on patient nutritional status of a FMD in patients with different cancer types and concomitant anticancer treatment. An amendment to the protocol (which includes the possibility of using imaging studies that are otherwise routinetly prescribed for disease monitoring, e.g. CT scans, for body composition evaluation) was approved by the Ethics Committee of the Regione Liguria on April 8th 2019 (#27). The FMD tested was described elesenwhere^{4,6}. Throughout the clinical study, patients have received dietary counseling for the intervals between FMD cycles, aiming at providing an appropriate intake of proteins (1.5 g/kg ideal weight/day, primarily from seafood and legumes), essential fatty acids, vitamins and minerals^{25,26} and have also been invited to perform light/moderate daily muscle training (e.g. 500-600 kJ/day; as allowed by their medical condition; https://docs.google.com/presentation/d/1szaRW4t-pZQI17o2Pe0747FsUEOVWWiA6BHja0p8H_0/edit#slide=id.g34ad7971 84_0_0) to enhance muscle anabolism⁴⁸. Between FMD cycles patients were prescribed amino acid supplements 11 (Aminotrofic; 30 g essential amino acids two times a day) in the presence of a difficulty in maintaining phase angle values of 5 degrees or greater. Additional information on this trial is available at: https://clinicaltrials.gov/ct2/show/NCT03595540. Patient serum for subsequent ELISA assays of circulating growth factors and adipokines has been routinely collected before commencement of the first and of the second FMD cycle. In addition, in those patients who live close to the enrolment site, blood draws were also performed on day 6 of the FMD at cycle. Blood glucose and ketone bodies were self-measured by the patients using Glucomen Areo 2k (Menarini).

The NCT03340935 clinical trial, was conducted at the Fondazione IRCCS Istituto Nazionale dei Tumori, Milan, Italy. This study is aimed at assessing the safety, feasibility and metabolic effects of a FMD in cancer patients treated with different standard antitumor therapies. Patients with any malignancy, with the exception of small cell neuroendocrine tumors, were considered for enrollment in this study. The FMD was administered up to a maximum of 8 consecutive cycles in combination with standard adjuvant treatments or therapies for advanced disease. The FMD utilized in this study consisted of a 5-day plant-based, low-calorie (600 Kcal on day 1, followed by 300 KCal/day on days 2 to 5), low-protein, low carbohydrate diet (patent pending). The FMD was repeated every 3 or 4 weeks up to a maximum of 8 consecutive cycles. Additional information on this trial can be obtained at: https: / /clinicaltrials.gov/ct2/ show/ NCT03340935. 2

### CT scan-based body composition analysis

CT scans of Pt.#1 and Pt.#3 were acquired with different CT scanners. Both 1.25-mm and 5-mm slice thickness with standard body kernel were available. The whole body was scanned from the lung apex to the pubic symphysis (Pt.#1). Reconstructed axial images with both a 1.25-mm and a 5-7 mm slice thickness were analysed using the software installed on the workstations of the IRCCS Ospedale Policlinico San Martino Radiology Department (Suite-Estensa 1.9-Ebit-Esaote Group Company, 2015). The third lumbar vertebra (L3), at the level in which both transverse processes are clearly visible, was the bony landmark for the estimation of total muscle area and subcutaneous fat. If abdominal CT scans were not available, but only thoracic CT was present (Pt.#3), estimation of total muscle area (red areas) and fat at the level of the Louis angle (manubriosternal joint that lies at the level of the second costal cartilage) at baseline and after repeated FMD cycles were done according to⁴⁹.

### Statistical Analysis

All in vitro data are represented as mean ± SD of at least three independent experiments. All in vivo data are represented as mean ± SEM. Statistical analyses were performed with GraphPad Prism software version 5 (GraphPad Software). All parameters were tested by paired t test or one-way ANOVA followed by Tukey's test. p values <0.05 were considered significant. To evaluate changes in fat-free body mass, fat body mass and phase angle over time, a linear mixed effects model was fitted taking into account absolute (values expressed as kgs) fat-free body mass, absolute fat body mass (values expressed as kgs) and phase angle (values expressed as degrees) as a function of time assessment, with a random covariate represented by subject ID (package lme4 in the R Environment for Statistical Computation). The following abbreviations were utilized in our figure legends to 1 define the degree of significance: ns: non-significant; *: p<0.05; **: p<0.01; ***: p<0.001.

### References:

1 DeVita, V. J., Laurence, TS. , Rosenberg, SA.. DeVita, Hellmann and Rosenberg's cancer: principles & practice of oncology. 11th edn, 1269-1316 (Wolters Kluwer, 2019).
2 Ferlay, J. et al. Cancer incidence and mortality worldwide: sources, methods and major patterns in GLOBOCAN 2012. International journal of cancer 136, E359-386, doi:10.1002/ijc.29210 (2015).
3 Araki, K. & Miyoshi, Y. Mechanism of resistance to endocrine therapy in breast cancer: the important role of PI3K/Akt/mTOR in estrogen receptor-positive, HER2-negative breast cancer. Breast cancer 25, 392-401, doi:10.1007/s12282-017-0812-x (2018).
4 Brandhorst, S. et al. A Periodic Diet that Mimics Fasting Promotes Multi-System Regeneration, Enhanced Cognitive Performance, and Healthspan. Cell metabolism 22, 86-99, doi:10.1016/j.cmet.2015.05.012 (2015).
5 Di Biase, S. et al. Fasting-Mimicking Diet Reduces HO-1 to Promote T Cell-Mediated Tumor Cytotoxicity. Cancer Cell 30, 136-146, doi:10.1016/j.ccell.2016.06.005 (2016).
6 Wei, M. et al. Fasting-mimicking diet and markers/risk factors for aging, diabetes, cancer, and cardiovascular disease. Sci Transl Med 9, doi:10.1126/scitranslmed.aai8700 (2017).
7 AlFakeeh, A. & Brezden-Masley, C. Overcoming endocrine resistance in hormone receptor-positive breast cancer. Current oncology 25, S18-S27, doi:10.3747/co.25.3752 (2018).
8 Lee, A. V., Cui, X. & Oesterreich, S. Cross-talk among estrogen receptor, epidermal growth factor, and insulin-like growth factor signaling in breast cancer. Clinical cancer research : an official journal of the American Association for Cancer Research 7, 4429s-4435s; discussion 4411s-4412s 21 (2001).
9 Sachs, N. et al. A Living Biobank of Breast Cancer Organoids Captures Disease Heterogeneity. Cell 172, 373-386 e310, doi:10.1016/j.cell.2017.11.010 (2018).
10 Jones, J. I. & Clemmons, D. R. Insulin-like growth factors and their binding proteins: biological actions. Endocrine reviews 16, 3-34, doi:10.1210/edrv-16-1-3 (1995).
11 Sanchez-Jimenez, F., Perez-Perez, A., de la Cruz-Merino, L. & Sanchez-Margalet, V. 1 Obesity and Breast Cancer: Role of Leptin. Frontiers in oncology 9, 596, doi:10.3389/fonc.2019.00596 (2019).
12 Garofalo, C., Sisci, D. & Surmacz, E. Leptin interferes with the effects of the antiestrogen ICI 182,780 in MCF-7 breast cancer cells. Clinical cancer research : an official journal of the American Association for Cancer Research 10, 6466-6475, doi:10.1158/1078-0432.CCR-04-0203 (2004). 7
13 Bougaret, L. et al. Adipocyte/breast cancer cell crosstalk in obesity interferes with the anti-8 proliferative efficacy of tamoxifen. PloS one 13, e0191571, doi:10.1371/journal.pone.0191571 (2018).
14 Hopkins, B. D. et al. Suppression of insulin feedback enhances the efficacy of PI3K inhibitors. Nature 560, 499-503, doi:10.1038/s41586-018-0343-4 (2018).
15 Pollak, M. The insulin and insulin-like growth factor receptor family in neoplasia: an update. Nature reviews. Cancer 12, 159-169, doi:10.1038/nrc3215 (2012).
16 Jarde, T., Perrier, S., Vasson, M. P. & Caldefie-Chezet, F. Molecular mechanisms of leptin and adiponectin in breast cancer. European journal of cancer 47, 33-43, doi:10.1016/j.ejca.2010.09.005 (2011).
17 Saxena, N. K. et al. Concomitant activation of the JAK/STAT, PI3K/AKT, and ERK signaling is involved in leptin-mediated promotion of invasion and migration of hepatocellular carcinoma cells. Cancer research 67, 2497-2507, doi:10.1158/0008-5472.CAN-06-3075 (2007).
18 Cristofanilli, M. et al. Fulvestrant plus palbociclib versus fulvestrant plus placebo for treatment of hormone-receptor-positive, HER2-negative metastatic breast cancer that progressed on previous endocrine therapy (PALOMA-3): final analysis of the multicentre, double-blind, phase 3 randomised controlled trial. The Lancet. Oncology 17, 425-439, doi:10.1016/S1470-2045(15)00613-0 (2016).
19 Lasham, A. et al. A novel EGR-1 dependent mechanism for YB-1 modulation of paclitaxel response in a triple negative breast cancer cell line. International journal of cancer 139, 1157-1170, doi:10.1002/ijc.30137 (2016).
20 Shajahan-Haq, A. N. et al. EGR1 regulates cellular metabolism and survival in endocrine resistant breast cancer. Oncotarget 8, 96865-96884, doi:10.18632/oncotarget.18292 (2017).
21 Di Biase, S. et al. Fasting regulates EGR1 and protects from glucose- and dexamethasone-dependent sensitization to chemotherapy. PLoS Biol 15, e2001951, doi:10.1371/journal.pbio.2001951 (2017).
22 Di Leva, G. et al. Estrogen mediated-activation of miR-191/425 cluster modulates tumorigenicity of breast cancer cells depending on estrogen receptor status. PLoS Genet 9, e1003311, doi:10.1371/journal.pgen.1003311 (2013).
23 Hawley, S. A. et al. Phosphorylation by Akt within the ST loop of AMPK-alpha1 down-regulates its activation in tumour cells. The Biochemical journal 459, 275-287, doi:10.1042/BJ20131344 (2014).
24 Ando, S., Barone, I., Giordano, C., Bonofiglio, D. & Catalano, S. The Multifaceted Mechanism of Leptin Signaling within Tumor Microenvironment in Driving Breast Cancer Growth and Progression. Frontiers in oncology 4, 340, doi:10.3389/fonc.2014.00340 (2014).
25 Arends, J. et al. ESPEN guidelines on nutrition in cancer patients. Clin Nutr 36, 11-48, doi:10.1016/j.clnu.2016.07.015 (2017).
26 Arends, J. et al. ESPEN expert group recommendations for action against cancer-related malnutrition. Clin Nutr 36, 1187-1196, doi:10.1016/j.clnu.2017.06.017 (2017).
27 Grundmann, O., Yoon, S. L. & Williams, J. J. The value of bioelectrical impedance analysis and phase angle in the evaluation of malnutrition and quality of life in cancer patients--a comprehensive review. European journal of clinical nutrition 69, 1290-1297, doi:10.1038/ejcn.2015.126 (2015).
28 Turner, N. C. et al. Palbociclib in Hormone-Receptor-Positive Advanced Breast Cancer. The New England journal of medicine 373, 209-219, doi:10.1056/NEJMoa1505270 (2015).
29 Creighton, C. J. et al. Insulin-like growth factor-I activates gene transcription programs strongly associated with poor breast cancer prognosis. Journal of clinical oncology : official journal of the American Society of Clinical Oncology 26, 4078-4085, doi:10.1200/JCO.2007.13.4429 (2008).
30 Karey, K. P. & Sirbasku, D. A. Differential responsiveness of human breast cancer cell lines MCF-7 and T47D to growth factors and 17 beta-estradiol. Cancer research 48, 4083-4092 (1988).
31 Baselga, J. et al. Everolimus in postmenopausal hormone-receptor-positive advanced breast cancer. The New England journal of medicine 366, 520-529, doi:10.1056/NEJMoa1109653 (2012).
32 Andre, F. et al. Alpelisib for PIK3CA-Mutated, Hormone Receptor-Positive Advanced Breast Cancer. The New England journal of medicine 380, 1929-1940, doi:10.1056/NEJMoa1813904 (2019).
33 Hu, R., Hilakivi-Clarke, L. & Clarke, R. Molecular mechanisms of tamoxifen-associated endometrial cancer (Review). Oncology letters 9, 1495-1501, doi:10.3892/ol.2015.2962 (2015).
34 Piacente, F. et al. Nicotinic Acid Phosphoribosyltransferase Regulates Cancer Cell Metabolism, Susceptibility to NAMPT Inhibitors, and DNA Repair. Cancer research 77, 3 3857-3869, doi:10.1158/0008-5472.CAN-16-3079 (2017).
35 Caffa, I. et al. Fasting potentiates the anticancer activity of tyrosine kinase inhibitors by strengthening MAPK signaling inhibition. Oncotarget 6, 11820-11832, doi:10.18632/oncotarget.3689 (2015).
36 Ciribilli, Y. et al. The coordinated p53 and estrogen receptor cis-regulation at an FLT1 promoter SNP is specific to genotoxic stress and estrogenic compound. PloS one 5, e10236, doi:10.1371/journal.pone.0010236 (2010).
37 Liu, C. Y. et al. Tamoxifen induces apoptosis through cancerous inhibitor of protein phosphatase 2A-dependent phospho-Akt inactivation in estrogen receptor-negative human breast cancer cells. Breast cancer research : BCR 16, 431, doi:10.1186/s13058-014-0431-9 (2014).
38 Massarweh, S. et al. Tamoxifen resistance in breast tumors is driven by growth factor receptor signaling with repression of classic estrogen receptor genomic function. Cancer research 68, 826-833, doi:10.1158/0008-5472.CAN-07-2707 (2008).
39 Mishra, A. K., Abrahamsson, A. & Dabrosin, C. Fulvestrant inhibits growth of triple negative breast cancer and synergizes with tamoxifen in ERalpha positive breast cancer by up-regulation of ERbeta. Oncotarget 7, 56876-56888, doi: 10.18632/oncotarget. 10871 (2016).
40 Ikeda, H. et al. Combination treatment with fulvestrant and various cytotoxic agents (doxorubicin, paclitaxel, docetaxel, vinorelbine, and 5-fluorouracil) has a synergistic effect in estrogen receptor-positive breast cancer. Cancer science 102, 2038-2042, doi:10.1111/j.1349-7006.2011.02050.x (2011).
41 Massarweh, S. et al. Mechanisms of tumor regression and resistance to estrogen deprivation 1 and fulvestrant in a model of estrogen receptor-positive, HER-2/neu-positive breast cancer. Cancer research 66, 8266-8273, doi:10.1158/0008-5472.CAN-05-4045 (2006).
42 Vijayaraghavan, S. et al. CDK4/6 and autophagy inhibitors synergistically induce senescence in Rb positive cytoplasmic cyclin E negative cancers. Nature communications 8, 5916, doi:10.1038/ncomms15916 (2017).
43 Cook Sangar, M. L. et al. Inhibition of CDK4/6 by Palbociclib Significantly Extends Survival in Medulloblastoma Patient-Derived Xenograft Mouse Models. Clinical cancer research : an official journal of the American Association for Cancer Research 23, 5802-5813, doi:10.1158/1078-0432.CCR-16-2943 (2017).
44 Michaloglou, C. et al. Combined Inhibition of mTOR and CDK4/6 Is Required for Optimal Blockade of E2F Function and Long-term Growth Inhibition in Estrogen Receptor-positive Breast Cancer. Molecular cancer therapeutics 17, 908-920, doi:10.1158/1535-7163.MCT-17-0537 (2018).
45 Lee, C. et al. Reduced levels of IGF-I mediate differential protection of normal and cancer cells in response to fasting and improve chemotherapeutic index. Cancer research 70, 1564-1572, doi:10.1158/0008-5472.CAN-09-3228 (2010).
46 Ahima, R. S. et al. Role of leptin in the neuroendocrine response to fasting. Nature 382, 250-252, doi:10.1038/382250a0 (1996).
47 Lee, C. et al. Fasting cycles retard growth of tumors and sensitize a range of cancer cell types to chemotherapy. Sci Transl Med 4, 124ra127, doi:10.1126/scitranslmed.3003293 (2012).
48 Reidy, P. T. et al. Protein blend ingestion following resistance exercise promotes human muscle protein synthesis. The Journal of nutrition 143, 410-416, doi:10.3945/jn.112.168021 (2013).
49 Rossi, F., Valdora, F., Barabino, E., Calabrese, M. & Tagliafico, A. S. Muscle mass estimation on breast magnetic resonance imaging in breast cancer patients: comparison between psoas muscle area on computer tomography and pectoralis muscle area on MRI. European radiology 29, 494-500, doi:10.1007/s00330-018-5663-0 (2019).

## Claims

1. A therapeutic combination of a compound having antiestrogenic activity and a CDK4/6 inhibitor for use in a method for the treatment of breast cancer (BC) or other estrogen-responsive tumors in a human patient, wherein the method comprises subjecting said patient to reduced calorie intake for a period of 24-190 hours while said patient is being treated with said therapeutic combination, wherein said compound having antiestrogenic activity is fulvestrant and said CDK4/6 inhibitor is Palbociclib, and wherein said reduced calorie intake is a daily calorie intake reduced by 50-100%, more preferably by 75-100%, with respect to the normal daily calorie intake, which is 2000-3000 kcal/day for an adult male subject and 1600-2400 kcal/day for an adult female subject.

2. The therapeutic combination for the use according to claim 1, wherein said patient is fed with foods with a high content of monounsaturated and polyunsaturated fats and a reduced content of proteins and carbohydrates (≥ 50% of the calories coming from said fats).

3. The therapeutic combination for the use according to claim 1 or 2, wherein said period of reduced calorie intake is of 48 to 150 hours, preferably 120 hours.

4. The therapeutic combination for the use according to any one of claims 1 to 3, wherein said period of reduced calorie intake with concurrent administration of the compound having antiestrogenic activity and of the CDK4/6 inhibitor to the patient is repeated one or more times after respective periods of 5-60 days, during which said patient is given the compound having antiestrogenic activity and the CDK4/6 inhibitor while following a diet involving a normal calorie intake.

5. The therapeutic combination having antiestrogenic activity for the use according to any one of claims 1 to 4, wherein said reduced calorie intake is obtained by fasting or by means of dietetic food with reduced calorie and/or protein content but containing all necessary micronutrients to prevent malnutrition.

6. A pharmaceutical composition comprising fulvestrant, palbociclib and a pharmaceutically acceptable carrier, for use in a method for the treatment as defined in any one of claims 1-5.

## Patentansprüche

1. Eine therapeutische Kombination aus einer Verbindung mit antiöstrogener Aktivität und einem CDK4/6-Inhibitor zur Verwendung in einem Verfahren zur Behandlung von Brustkrebs (BC) oder anderen östrogenresponsiven Tumoren bei einem menschlichen Patienten, wobei das Verfahren umfasst, dass der Patient einer reduzierten Kalorienzufuhr für einen Zeitraum von 24 bis 190 Stunden unterzogen wird, während der Patient mit der therapeutischen Kombination behandelt wird,
wobei die Verbindung mit antiöstrogener Aktivität Fulvestrant ist und der CDK4/6-Inhibitor Palbociclib ist,
und wobei die reduzierte Kalorienzufuhr eine tägliche Kalorienaufnahme ist, die im Vergleich zur normalen täglichen Kalorienaufnahme um 50 bis 100 %, vorzugsweise um 75 bis 100 %, reduziert ist,
wobei die normale tägliche Kalorienaufnahme 2000 bis 3000 kcal/Tag für einen erwachsenen männlichen Probanden und 1600 bis 2400 kcal/Tag für einen erwachsenen weiblichen Probanden beträgt.

2. Die therapeutische Kombination zur Verwendung gemäß Anspruch 1, wobei der Patient mit Lebensmitteln ernährt wird, die einen hohen Gehalt an einfach und mehrfach ungesättigten Fettsäuren und einen reduzierten Gehalt an Proteinen und Kohlenhydraten aufweisen (≥ 50 % der Kalorien stammen aus diesen Fetten).

3. Die therapeutische Kombination zur Verwendung gemäß Anspruch 1 oder 2, wobei der Zeitraum der reduzierten Kalorienzufuhr 48 bis 150 Stunden, vorzugsweise 120 Stunden, beträgt.

4. Die therapeutische Kombination zur Verwendung gemäß einem der Ansprüche 1 bis 3, wobei der Zeitraum der reduzierten Kalorienzufuhr mit gleichzeitiger Verabreichung der Verbindung mit antiöstrogener Aktivität und des CDK4/6-Inhibitors an den Patienten ein- oder mehrmals nach jeweiligen Zeiträumen von 5 bis 60 Tagen wiederholt wird, während derer der Patient die Verbindung mit antiöstrogener Aktivität und den CDK4/6-Inhibitor erhält und einer Diät mit normaler Kalorienzufuhr folgt.

5. Die therapeutische Kombination zur Verwendung gemäß einem der Ansprüche 1 bis 4, wobei die reduzierte Kalorienzufuhr durch Fasten oder durch diätetische Nahrung mit reduziertem Kalorien- und/oder Proteingehalt erreicht wird, die jedoch alle notwendigen Mikronährstoffe enthält, um eine Mangelernährung zu verhindern.

6. Eine pharmazeutische Zusammensetzung, umfassend Fulvestrant, Palbociclib und einen pharmazeutisch akzeptablen Träger, zur Verwendung in einem Verfahren zur Behandlung gemäß einem der Ansprüche 1 bis 5.

## Revendications

1. Combinaison thérapeutique d'un composé ayant une activité anti-œstrogènes et d'un inhibiteur de CDK4/6 pour une utilisation dans un procédé pour le traitement du cancer du sein (CS) ou d'autres tumeurs sensibles aux œstrogènes chez un patient humain, où le procédé comprend le fait de soumettre ledit patient à un apport calorique réduit pendant une période de 24 à 190 heures tandis que ledit patient est traité avec ladite combinaison thérapeutique, où ledit composé ayant une activité anti-œstrogènes est le fulvestrant et ledit inhibiteur de CDK4/6 est le palbociclib, et où ledit apport calorique réduit est un apport calorique quotidien réduit de 50 à 100 %, de préférence encore de 75 à 100 %, par rapport à l'apport calorique quotidien normal, qui est de 2000 à 3000 kcal/jour pour un sujet masculin adulte et de 1600 à 2400 kcal/jour pour un sujet féminin adulte.

2. Combinaison thérapeutique pour l'utilisation selon la revendication 1, où ledit patient est nourri avec des aliments à haute teneur en graisses monoinsaturées et polyinsaturées et à teneur réduite en protéines et en glucides (≥ 50 % des calories provenant desdites graisses).

3. Combinaison thérapeutique pour l'utilisation selon la revendication 1 ou 2, où ladite période d'apport calorique réduit est de 48 à 150 heures, de préférence de 120 heures.

4. Combinaison thérapeutique pour l'utilisation selon l'une quelconque des revendications 1 à 3, où ladite période d'apport calorique réduit avec administration simultanée du composé ayant une activité anti-œstrogènes et de l'inhibiteur de CDK4/6 au patient est répétée une ou plusieurs fois après des périodes respectives de 5 à 60 jours, pendant lesquelles ledit patient reçoit le composé ayant une activité anti-œstrogènes et l'inhibiteur de CDK4/6 tout en suivant un régime impliquant un apport calorique normal.

5. Combinaison thérapeutique ayant une activité anti-œstrogènes pour l'utilisation selon l'une quelconque des revendications 1 à 4, où ledit apport calorique réduit est obtenu par le jeûne ou au moyen d'aliments diététiques à teneur réduite en calories et/ou en protéines mais contenant tous les micronutriments nécessaires pour prévenir la malnutrition.

6. Composition pharmaceutique comprenant du fulvestrant, du palbociclib et un véhicule pharmaceutiquement acceptable, pour une utilisation dans un procédé pour le traitement tel que défini dans l'une quelconque des revendications 1 à 5.
